# EUROPEAN PATENT SPECIFICATION

(11) **EP 3 030 903 B1**
(45) Date of publication and mention of the grant of the patent: **20.02.2019**
(21) Application number: 14792541.6
(22) Date of filing: 07.08.2014
(51) Int. Cl.: G01N 33/50, B01L 3/00, G01N 33/543

(54) **METHOD FOR THE ANALYSIS OF IMMUNOREACTIVITY, AND A DEVICE SUITABLE FOR CARRYING OUT THE METHOD**
VERFAHREN ZUR IMMUNREAKTIVITÄTSANALYSE UND VORRICHTUNG ZUR DURCHFÜHRUNG DIESES VERFAHRENS
PROCÉDÉ POUR L'ANALYSE DE L'IMMUNORÉACTIVITÉ, ET DISPOSITIF CONVENANT À LA MISE EN OEUVRE DE CE PROCÉDÉ

(30) Priority: 07.08.2013 HU P1300474
(43) Date of publication of application: 15.06.2016
(73) Proprietor: Eötvös Loránd Tudományegyetem, 1053 Budapest (HU); Magyar Tudományos Akadémia, 1051 Budapest (HU); Diagnosticum Gyártó és Forgalmazó Zártkörüen Müködö Részvénytàrsasàg, 1047 Budapest (HU)
(72) Inventor: PRECHL, József, 1113 Budapest (HU); PAPP, Krisztián, 1065 Budapest (HU); SZITTNER, Zoltán, 1026 Budapest (HU)
(74) Representative: Machytka-Frank, Daisy
(86) International application number: PCT/HU2014/000068
(87) International publication number: WO 2015/019120

(56) References cited:
- EP-A1- 2 208 531
- WO-A1-03/004160
- WO-A1-2004/083859
- WO-A1-2009/125227
- US-A- 5 204 525
- KRISZTIÃ N PAPP ET AL: "Life on a microarray: assessing live cell functions in a microarray format", CMLS CELLULAR AND MOLECULAR LIFE SCIENCES, BIRKHÄUSER-VERLAG, BA, vol. 69, no. 16, 4 March 2012 (2012-03-04) , pages 2717-2725, XP035087262, ISSN: 1420-9071, DOI: 10.1007/S00018-012-0947-Z
- MARVIN FRITZLER ET AL: "The Emergence of Multiplexed Technologies as Diagnostic Platforms in Systemic Autoimmune Diseases", CURRENT MEDICINAL CHEMISTRY, vol. 13, no. 21, 1 September 2006 (2006-09-01), pages 2503-2512, XP055162421, NL ISSN: 0929-8673, DOI: 10.2174/092986706778201639

## Description

### TECHNICAL FIELD

The present invention generally relates to capillary channel based antigen arrays, more particularly to systems and methods using live cells for the detection of interactions between antigens on an array, preferably on a microarray, and components of the sample, which is preferably a biological sample, e.g. a biological fluid contacted with the array.

Proteomics is the systematic study of the properties of the numerous protein components present in cells and organisms. The purpose of functional immunomics is to understand the highly diverse interactions between the molecular and cellular components of the immune system and integrate this knowledge into systems biological perspective of immunity. Protein arrays are tools for the study of the various interactions between macromolecules, especially proteins, and are used in proteomics and functional immunomics. Evidently, antigen arrays form a subset of protein microarrays. The characterization of the specificity of antibodies present in the blood is carried out by serological measurements. Diagnostic measurements (testing) carried out at or by the place of medical attendance are called 'point-of-care (POC)' testing or 'bed-side testing'. Within POC testing measurements those that can be implemented rapidly, without instruments or by simple devices, are called rapid diagnostic tests (RDT).

### BACKGROUND OF THE INVENTION

Contacting blood or other biological fluids with a collection of antigens results in the binding of antigen-specific antibodies circulating in the blood or other biological fluid to their target antigens. When antigens are bound to a support thereby forming an antigen array, optionally an antigen microarray, and the antigen array, preferably the antigen microarray, is contacted with a blood sample or other biological fluid, and the antibodies of the sample specifically binding to such antigens are detected, a binding pattern referred to as an antibody profile, which is characteristic of the tested sample, is generated (J.Prechl, K.Papp, and A.Erdei. 2010. Antigen microarrays: descriptive chemistry or functional immunomics? Trends Immunol. 31:133-137.). According to the state of the art, this binding pattern is revealed by detection (detector) antibodies labelled suitably to allow qualitative and quantitative detection of the immune complexes generated. Thus, the binding pattern indicates the antigens (i.e., the spots) of the antigen array where immune complexes are generated and the quantities thereof. A disadvantage of the detection method using detection antibodies is that in living organisms, effector functions elicited by immune complexes are controlled by all the constituents of the complex, including not one but all the antibody classes, complement components and potentially all other recognition molecules. Therefore, detection antibodies only provide indirect information regarding the biological functions of immune complexes, i.e., whether they are actually functional in the living biological environment. Detection antibodies recognise the tested antibodies (i.e. antigen-specific antibodies) on the basis of the antigenicity of said tested antibodies in the host animals used to generate such detection antibodies. This antigenicity is unrelated to the effector functions of the primary antibodies, which are exerted through the receptors of immune cells. Another problem is that detection antibodies recognise certain components of the immune complexes, only, e.g. a certain subclass of antibodies forming immune complexes, therefore, binding patterns, antibody profiles generated using detection antibodies indicate a segment of the entire immunoreactivity, only.

In the body, mainly monocytes and their progeny, and neutrophil granulocytes are responsible for recognising, analysing and removing immune complexes. They are equipped with an extensive panel of receptors which are able to bind to pathogens and self molecules, such as various antibodies and complement components. Monocytes, macrophages and dendritic cells are constantly monitoring the body and alerting other cells when potentially dangerous molecules are found. Neutrophil granulocytes migrating to the area of inflammation participate in the recognition and destruction of pathogens. These cells are therefore ideal as cellular sensors for probing antibody-antigen interactions. However, most mature cells bear some kind of receptor(s) for immune complexes, and are therefore also potentially suitable for this use.

Cells have been used on microarrays in a variety of formats and for different purposes. T lymphocytes bind to MHC-peptide complexes printed as arrays (Soen,Y., D.S.Chen, D.L.Kraft, M.M.Davis, and P.O.Brown, 2003, Detection and characterization of cellular immune responses using peptide-MHC microarrays. PLoS Biol 1:E65), leukaemia cells are captured by surface marker-specific capture antibodies (Belov,L., I.de, V, C.G.dos Remedios, S.P.Mulligan, and R.I.Christopherson, 2001, Immunophenotyping of leukaemias using a cluster of differentiation antibody microarray. Cancer Res 61:4483-4489), mesenchymal cells adhere to peptides of extracellular matrix components (Okochi,M., S.Nomura, C.Kaga, and H.Honda, 2008, Peptide array-based screening of human mesenchymal stem cell-adhesive peptides derived from fibronectin type III domain. Biochem Biophys Res Commun 371:85-89), and hepatocytes adhere to glycans (Nimrichter,L., A.Gargir, M.Gortler, R.T.Altstock, A.Shtevi, O.Weisshaus, E.Fire, N.Dotan, and R.L.Schnaar, 2004, Intact cell adhesion to glycan microarrays. Glycobiology 14:197-203), just to mention a few types of binding.

Monocytes carry a number of receptors for antibodies (IgG, IgA, IgM and IgE Fc receptors) and for complement components. These receptors can induce activation of the cell and mediate firm adhesion (Ehlers,M.R. 2000, CR3: a general purpose adhesion-recognition receptor essential for innate immunity. Microbes Infect. 2:289-294; Vidarsson, G. and J.G.van de Winkel, 1998, Fc receptor and complement receptor-mediated phagocytosis in host defence. Curr. Opin. Infect. Dis. 11:271-278). The number of the cells on the array can be revealed by fluorescent labelling and laser scanning (Belov et al., see above) or without labelling, by microscopy (Soen et al., see above).

Serological rapid diagnostic tests are based on detecting changes which are due to specific binding of antigen(s) to antibody(ies) and which are easy to recognise and/or to measure. Such changes include e.g. agglutination, precipitation, colour reaction with labelled reagents. Serological rapid diagnostic tests currently used operate in cell-free manner, the ability of cells to recognise immune complexes is not utilized.

In US 5,204,525 a capillary flow device is disclosed, which has capillary channels containing microparticles to which antibodies are conjugated, and the device further has a capillary. In addition, the device has incubation chambers being arranged between the capillary channels and the capillary.

In EP2208531 A1 a capillary microfluid channel is disclosed which comprises a first capillary channel, an adjacent collection chamber having a waste outlet, and a detection chamber connected to the collection chamber through a washing chamber comprising paramagnetic particles.

In WO 2009/125227 A1 an assay device is disclosed which has a test flow path formed by a first capillary channel and a reference flow path formed by a second capillary channel, wherein both paths start from the same sample application region and both paths end in the same indicator region. A method of performing an assay using gas generating means in the device is also disclosed.

In WO 03/004160 A1 a microfluidic electrochemical assay apparatus and method are disclosed. An agglutination based sample testing device is disclosed in WO 2004/083859 A1.

The review article by K.N.Papp et al. (Cell Mol Life Sci, 2012; 69(16):2717-25) describes cell-based array technology as permitting simultaneous detection of several different cell surface molecules, allowing the complex characterization of cells. The review summarizes possible applications of cell-based arrays.

The article by M. Fritzler et al. (Curr Med Chem, 2006; 13(21):2503-12) reviews new technologies that are capable of identifying an increasing spectrum of autoantibodies and other biomarkers in autoimmune diseases, including cell and tissue arrays, line immunoassays, addressable laser bead immunoassays, microarrays in microfluidic platforms and nanobarcode particles.

### DESCRIPTION OF THE INVENTION

It is an object of the present invention to provide a method for the analysis (i.e. testing) of immunoreactivity, as defined in the claims which method, when compared to the methods known in the art, better reflects the biological functions of the detected immune complexes and immunoreactivity and whether such immune complexes are actually functional in the living biological environment. A further object of the invention is to provide a novel method and a novel device useful in diagnostics, as defined in the claims which, when compared to known technical solutions, better reflect the biological effects of the detected antibodies on cells, and provide unambiguous, semi-quantitative, multiplex results of measurement.

A fundamental observation providing basis for the present invention is that the above object can be achieved by using cells, preferably adherent cells, e.g. immune cells, e.g. monocytes, macrophages, dendritic cells, neutrophil granulocytes. Cells act as biosensors when detecting the immune complex patterns which are generated on the antigen array, preferably on antigen microarray, and which reflect the reactivity of the humoral immune system. Under suitable conditions, the cells able to adhere (adherent cells) and having specific receptors to the components of immune complexes adhere to the surface of the antigen array, which is preferably a microarray, in a manner ensuring that the number and distribution of the adhered cells depend on the quantity and quality (immunoglobulin isotypes and complement product types) of the immune complexes generated on that surface and characterise them. We have found that antibodies and complement components in the immune complexes activate cells and enhance their adherence to an extent that comparing the areas of the surface bearing biologically important immune complexes with those areas of the surface that were treated with a biological fluid but not comprising immune complexes at all or comprising immune complexes of insignificant biological relevance, the difference between said areas concerning the extent of cell activation and cell adhesion is observable or measurable and can be used to generate patterns.

It has been found that immune cells, preferably bone marrow-derived phagocyte cells, most preferably monocytes, macrophages and neutrophil granulocytes, wherein the immune cells are optionally labelled, are more useful than detection antibodies for distinguishing immune complexes with various biological properties on antigen arrays because cells are more efficiently activated by and adhere more efficiently to immune complexes comprising the tested antibodies and having a higher immunological activity, as a result of the changes in their adherence. In contrast to detection antibodies, cells integrate all biologically relevant information, are superior in reflecting biological functions of antigen array-bound antibodies and represent a relatively simple but robust alternative for profiling immune responses, e.g. for profiling interactions between proteins responsible for humoral immunoreactivity and antigens.

Applying cells to antigen arrays, preferably to antigen microarrays, results in localized responses of the cells, corresponding to the antigen array (preferably antigen microarray) features.

We have found that the adhesion of cells depends on the quantity of IgG present in the immune complex and on the relative IgG subclass composition of the antibodies captured. It has been found that cells, preferably monocyte cell lines and/or neutrophil granulocytes prefer certain subclasses to others when adhering, giving the following pattern: IgG3>IgG1>IgG4>IgG2. This finding confirms that IgG subclasses responsible for immunological effector functions can be detected more efficiently, that is, the binding pattern carries biologically relevant information.

In addition, it has been found that the adhesion of the cells is primarily mediated by IgG Fc receptors.

Accordingly, it has also been found that cellular detection can be used to determine not only the presence of antigen-specific antibodies but also a function thereof, namely, that the immune complexes formed by them bind to the IgG Fc receptors of the cell. IgG Fc receptors play a key role in the regulation of immune cell functions including e.g. phagocyte activation, stimulation of cytokine production and initiation of inflammatory processes. Thus, cellular detection indicates that the body can produce such responses against the antigen in question.

We have further found that the adherence initiated by the IgG Fc receptors of the cell is strong enough to resist the liquid (i.e. fluid) flow shearing forces e.g. generated during washing steps or by capillary forces and/or by external action. When flowing cells on a surface coated by IgG antibodies of suitable subclass(es), the flow slows down measurably. Further, it has been found that immobilising antigens on the surface of capillaries of proper diameter, and then flowing blood or diluted blood comprising antigen-specific antibodies, binding of antibodies takes place and then adherence of cells, thereby causing a measurably slowing down of the flow of the blood or diluted blood.

On the basis of the above detailed findings we developed new methods useful in diagnostics and devices suitable for implementation, preferably rapid implementation of the methods (e.g. diagnostic methods). The novel devices according to the invention include capillary channel systems (microfluidic systems). The term 'capillary channel' includes any channel in which capillary forces act. The microfluidic devices and capillary channel systems according to the invention are particularly suitable for carrying out the methods of the invention.

The invention provides methods, uses and devices, e.g. those useful in diagnostics, as defined in the main claims. The dependent claims describe certain preferred embodiments according to the invention.

The invention provides a method for the analysis of immunoreactivity on an antigen array, the method comprises contacting with a sample an antigen array comprising one or more antigens and optionally one or more reference substances; and detecting by cells the complexes formed by the one or more antigens and by one or more components of the sample which may be captured by the antigens and, wherein for the detecting by cells the sample serves as a source of the cells, or the cells are from a source other than the sample, the step of detecting by cells comprises contacting the array with cells, namely the method comprises introducing a sample into a capillary channel system as defined in claim 1, i.e. into a capillary channel system which comprises
- a measuring channel (106), which measuring channel (106) is a capillary channel comprising
   o a trap section (1, 22, 38, 40, 42, 44, 46, 47, 48) having a coating comprising an antigen, the trap section (1, 22, 38, 40, 42, 44, 46, 47, 48) enables the adhesion of cells of a sample,
   ∘ a collector section (2, 20) having a volume which is at least as large as that of the trap section (1, 22, 38, 40, 42, 44, 46, 47, 48), and being adapted for receiving sample flowing through the trap section (1, 22, 38, 40, 42, 44, 46, 47, 48), and
   ∘ a reader section (3, 18) adjacent to the collector section (2, 20),
   wherein the collector section (2, 20) is located in the part between the trap section (1, 22, 38, 40, 42, 44, 46, 47, 48) and the reader section (3, 18) of the capillary channel, and

- a negative control channel (10, 104) having a structural configuration identical with that of the measuring channel (106) and being provided with a coating which differs from that of the measuring channel (106) or being formed without coating, and after the liquid front of the sample in the negative control channel having reached the reader section, observing or measuring on the reader sections of the -individual channels a feature of the flow of the sample, preferably the flow rate of the sample or a quantity proportional thereto.

As described the step of detecting by cells can comprise one or more of the following steps i) to v), thereby obtaining data for the areas of the individual features of the array:
i) revealing the cells adhered on the areas of the individual features of the antigen array,
ii) determining the amount of cells adhered on the areas of the individual features of the antigen array,
iii) measuring or observing a quantity proportional to the amount of cells adhered on the areas of the individual features of the antigen array,
iv) measuring cell adhesion on the areas of the individual features of the antigen array,
v) measuring cell activation of the cells adhered on the areas of the individual features of the antigen array.

In preferred embodiments of the methods of the invention the analysis of immunoreactivity is aimed at the diagnosis of a condition or disorder accompanied by the presence of one or more specific antibodies, and the array comprises at least one antigen capable to bind to said one or more specific antibodies, and optionally the method further comprises comparing the data observed or measured for the areas of the individual antigens and for the areas of the individual reference substances.

The disclosure further provides a method for the analysis of immunoreactivity on an antigen array, the method comprises
a) contacting with a sample an antigen array comprising one or more antigens and optionally one or more reference substances,
b) optionally carrying out a washing step,
c) contacting the array with cells, wherein the sample serves as a source of the cells, or the cells are from a source other than the sample,
d) revealing the cells adhered on the areas of the individual features of the antigen array, determining the amount of cells adhered on the areas of the individual features of the antigen array, measuring a quantity proportional to the amount of cells adhered on the areas of the individual features of the antigen array, measuring cell adhesion on the areas of the individual features of the antigen array,
   and/or measuring cell activation of the cells adhered on the areas of the individual features of the antigen array, and
e) optionally comparing the data observed or measured for the areas of the individual antigen(s) and for the areas of the individual reference substance(s), namely the method comprises introducing a sample into a capillary channel system as defined in claim 1, and after the liquid front of the sample in the negative control channel having reached the reader section of the capillary channel system observing or measuring on the reader sections of the individual channels a feature of the flow of the sample, preferably the flow rate of the sample or a quantity proportional thereto.

Also provided is a method for the qualitative and/or semi-quantitative analysis of the composition of one or more immune complexes on an antigen array, the method comprises contacting with cells the antigen array comprising the one or more immune complexes, wherein for the contacting with cells either the sample serves as a source of the cells, or the cells are from a source other than the sample, and detecting the cells adhered to the one or more immune complexes, determining the amount of cells adhered, measuring a quantity proportional to the amount of cells adhered, measuring cell adhesion on the one or more immune complexes and/or measuring cell activation of the cells adhered.

A capillary channel is also provided, which comprises a trap (also called as trap section) having a coating, preferably an antigen coating, and further comprises a collector section (also called as collector or collector container or collector container section) having a volume which is at least as large as that of the trap section, and a reader section (also called as reader) adjacent to the collector section, wherein the reader section optionally comprises one or more physical and/or chemical sensors adapted for measuring a flow rate and/or one or more markings for visual reading of a liquid front (i.e. fluid front) position, and wherein the collector section is located in the part between the trap section and the reader section of the capillary channel.

The invention also provides a capillary channel system as defined in claim 1.

The invention also provides a microfluidic device comprising a capillary channel system according to the invention, as defined in the claims, wherein the capillary channel system is formed in the material or materials constituting the device.

The invention provides also a method for the analysis of immunoreactivity, comprising introducing a sample into a capillary channel system according to the invention, and after the liquid front of the sample in the negative control channel having reached the reader section, and, preferably, after both the liquid front of the sample in the antigen-containing capillary channel(s) and the liquid front of the sample in the negative control channel having reached the reader section, observing or measuring on the reader sections of the individual channels a feature of the flow of the sample, preferably the flow rate of the sample or a quantity proportional thereto.

In a preferred embodiment of the method of the invention, the analysis of immunoreactivity is aimed at the diagnosis of a condition or disorder accompanied by the presence of one or more specific antibodies, and the trap section of at least one measuring channel comprises an antigen capable to bind to at least one of said specific antibodies, and the method optionally further comprises comparing the flow features observed or measured in the individual channels, wherein the flow features are preferably the flow rates.

Kits are also provided, e.g. diagnostic kits, comprising a support suitable for creating antigen arrays, preferably antigen microarrays, one or more antigens and, optionally, one or more reference substances, all preferably arranged as array features on the solid support; the kits preferably comprise cells, preferably monocytes, optionally a washing liquid; optionally a buffer for diluting the sample; optionally a substance suitable for cell labelling or labelled cells. In the case of one-dimensional linear antigen arrays, preferably antigen microarrays, the support suitable for creating the antigen array, preferably antigen microarray, is preferably a capillary; for two-dimensional antigen arrays, preferably antigen microarrays, the support is preferably a plate. Optionally, the kit may comprise written and/or pictorial instructions for the use of the kits.

The methods, capillary channel systems, microfluidic devices and kits according to the invention and disclosure, respectively are preferably used in the diagnosis of a condition or a disorder accompanied by the presence of one or more specific antibodies, preferably in the diagnosis of systemic lupus erythematosus, hepatitis B infection or immunity.

The kits can be used in the methods of the present invention and/or for the detection of a disease or condition related to immune profile changes.

Preferably, the capillary channel system or microfluidic device according to the invention is used as a rapid diagnostic device for point-of-care serological tests. Antigens applied to a solid support in the form of antigen arrays, preferably in the form of antigen microarrays are used for identifying targets of samples, preferably biological samples, e.g. biological fluids such as serum antibodies, and for generating binding profiles.

In the methods, devices and uses of the invention the antigen array is a capillary channel system according to the invention.

On the basis of the above, also provided the use of cells for the analysis of immunoreactivity on an antigen array, for the detection of immune complexes on an antigen array, or for the qualitative and/or semi-quantitative analysis of the composition of immune complexes on an antigen array.

### BRIEF DESCRIPTION OF THE DRAWINGS

In the following, certain exemplary technical solutions are illustrated by drawings.
Figure 1 shows the result of the detection of different antibody subclasses by detection antibodies to human IgG, complement C3 fixing ability and adhesion of U937 monocytoid cells.
Figure 2 shows the detection of the binding patterns of serum antibodies by detection antibodies to human IgG and by U937 monocytoid cells, as well as by IgG subclass-specific detection antibodies.
Figure 3 outlines the principle of the technical solution of the prior art (A) and that of the disclosure (B).
Figure 4 demonstrates that cell adhesion can be inhibited by blocking (IgGBL) the interaction between IgGs and their receptors.
Figure 5 shows the use of monocytes purified from human blood as detector cells.
Figure 6 shows the use of an antigen array created in the form of a capillary.
Figure 7 shows the identifiers of the antigens present on the SLE chip according to Example 6, in accordance with the arrangement of the array. The microarray carrying autoantigens and reference substances was reacted with the serum of a patient with systemic lupus erythematosus (SLE), then fluorescently labelled anti-IgG detection antibodies and monocytoid cells, respectively, were used for the detection of the immune complexes formed, that is, to reveal the binding patterns.
Figure 8 shows the SLE chip as detected by anti-IgG detection antibody.
Figure 9 shows the SLE chip as detected by U937 monocytoid cells.
Figure 10 is the graphical representation of the data from the SLE chip as detected _ by anti-IgG detection antibody.
Figure 11 is the graphical representation of the data from the SLE chip as detected by U937 monocytoid cells.
Figure 12 is a schematic cross-sectional view of a preferred embodiment of the capillary channel of the capillary channel system according to the invention.
Figure 13 illustrates the binding of antibodies and cell adhesion in a trap used according to the invention.
Figure 14 shows that in a capillary treated with a material (IgG3) which facilitates cell adhesion, the liquid flow slows down as compared to the liquid flow observed in a capillary treated with materials (albumin or, IgG2) that do not support cell adhesion.
Figure 15 illustrates an array of the capillary channels according to the invention.
Figure 16 illustrates the working of an array of capillary channels.
Figure 17 shows the result of the experiment carried out as described in Example 7.
Figure 18 illustrates the structure of the HBsAg test as described in Example 8.
Figure 19 is the chart of the results of the experiment performed as described in Example 8.
Figure 20 shows a part of the result of the experiment carried out as described in Example 8.
Figure 21 illustrates cross-sectional views of traps of preferred configuration.
Figure 22 illustrates cross-sectional views of two further traps of preferred configuration.
Figure 23A illustrates the test results of a sample with ++ positivity, in a reader section having a fixed negative control endpoint.
Figure 23B illustrates the test results of a sample with +++ positivity, in a reader section having a fixed measuring channel endpoint.
Figure 24A illustrates the test results of a negative sample in a reader section having a fixed negative control endpoint.
Figure 24B illustrates an invalid (not evaluable) test result in a reader section having a fixed measuring channel endpoint.
Figure 25 illustrates a negative test result.
Figure 26 shows the application of detection cells: by the application of human blood or washed neutrophil granulocytes from blood.
Figure 27 illustrates in a top view an embodiment of the capillary channel system according to the invention, the direction of flow is also indicated.
Figure 28 illustrates the configuration of the trap according to the embodiment according to Figure 27 in enlarged drawings.
Figure 29A illustrates a configuration of the trap in a further embodiment.
Figure 29B illustrates a configuration of the traversable cross sections in the trap according to the embodiment according to Figure 29A.
Figure 30A illustrates the treated chambers, according to Example 11.
Figures 30B and 30C show the result of the experiment carried out according to Example 11.
Figure 30D shows a Nexterion plate used in the experiments according to Examples 12 and 13. The place of the application of IgG3 and BSA solutions are indicated by arrows.
Figure 30E shows the result of the experiment carried out according to Example 12.
Figures 30F and 30G show the result of the experiment carried out according to Example 13.
Figure 30H illustrates a PDMS cast and a Nexterion plate fitted to each other and used in the experiments carried out according to Examples 12 and 13.

### MODES FOR CARRYING OUT THE INVENTION

After contacting the antigen array with the sample, immune complexes are allowed to form by the one or more antigens and by the one or more components of the sample which can be captured by said antigens.

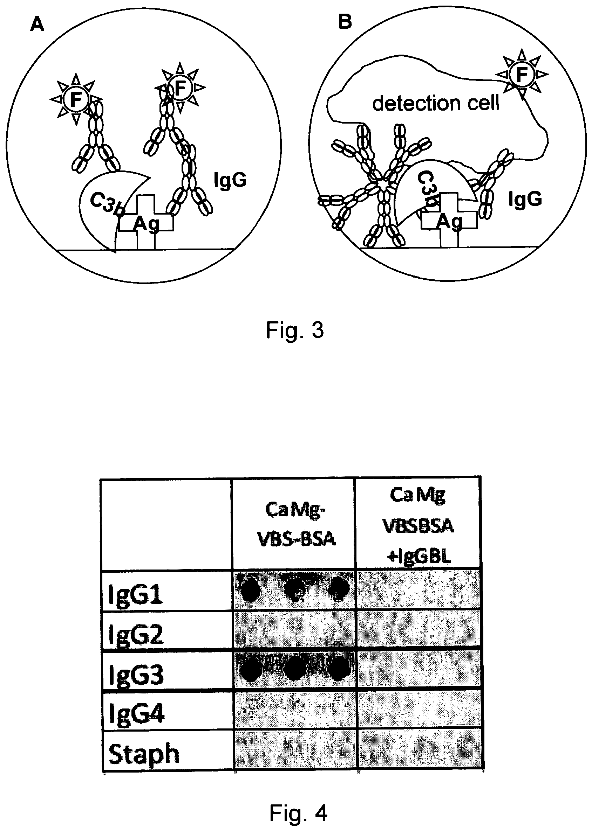

Antigens (Ag) are printed in an array format thereby forming an antigen array, preferably an antigen microarray, and are reacted with a sample, e.g. with a serum sample. A capillary channel system according to the invention is used as antigen array, the antigens are arranged, immobilized on a solid support, formed by the walls of the capillary channels. Immunoglobulins (e.g. IgG) and complement fragments (e.g. C3b) may bind to certain antigens of the antigen array (which is preferably an antigen microarray). In the prior art (Figure 3A), detection antibodies are used for detecting bound serum components.

According to the present invention (Figure 3B), cells (e.g. monocytes) are used for the detection, said cells act as sensor cells and are able to integrate multiple signals from all receptor-ligand interactions originating from the immune complex on the antigen array. Cell adhesion or activation can be utilised for read-out.

Immunoreactivity is the ability of the immune system to recognise, bind and influence a target by its soluble molecules or cells. The targets are typically antigens.

By the method of the invention typically the immunoreactivity of an individual is determined through testing a sample from said individual. The individual may be a human or an animal, e.g. a vertebrate animal such as a mammal or a bird.

In certain preferred embodiments of the methods of the disclosure, an immune profile is determined. Immune profile, as used herein, refers to the pattern obtainable through the determination of the reactivity against more than one antigens, i.e. a set of reactivities. The method of the present invention may form a part of a diagnostic method. In such diagnostic methods, the immune profile or immunoreactivity of a patient can be determined and may be compared to the immune profile or immunoreactivity of a healthy subject. Antigens are selected in a manner to generate an immune profile characteristic for the condition, e.g. disease to be diagnosed or excluded. The term 'diagnostic methods' includes also those methods which are directed e.g. to the analysis of immunity.

When the methods of the invention are intended to be used in diagnostics, it is preferable to measure samples already determined by other methods to show negative or positive reaction against one or more relevant antigens. That is, on the basis of measurement results for known positive samples and/or negative samples the method can be calibrated, i.e. calibration data can be obtained. Thus, the method disclosed may comprise the step of comparing the data obtained for the areas of the individual antigen-comprising array features with said calibration data.

### Antigens - Antigen arrays - Antibodies - Immune complexes

An 'antigen', as used herein, is a substance that in the organism of a vertebrate is capable of eliciting an immune response. The term 'antigens', as defined herein, includes, among others, molecules and moieties which stimulate the production and release of an antibody (or antibodies) that binds specifically to the them and/or which bind specifically to antibodies to form antigen/antibody complexes (immune complexes). In accordance with the present invention, antigens may be, but are not limited to, parasites, microbes, viruses, prions, components thereof, peptides, polypeptides, glycoproteins, proteins, lipids, glycolipids, carbohydrates, nucleic acids, DNA, RNA, small molecules, saccharides, allergens and autoantigens, combinations thereof, fractions thereof, or mimetics thereof.

An 'autoantigen' is a self antigen, which is a molecular component of the organism that responds to this particular molecule with antibody production.

In certain preferred embodiments of the invention, the one or more antigens are selected from those listed in Table 1 below. In further preferred embodiments the methods of the invention are used for the detection of a disease or condition listed in Table 1 and the respective one or more antigens are selected from those listed in Table 1 below.

The term 'antigen array', as used herein, refers to an ordered arrangement of antigens and, optionally, of reference substances, wherein the antigens and reference substances are on a solid support. The_antigen array is preferably an antigen microarray. The features are arranged in a manner to ensure that the array has at least one, more preferably at least two or more different array features, more preferably at least 10 array features, and most preferably at least 100 array features, and even more preferably at least 1,000, on a surface of a few cm². Methods for the detection of macromolecular interactions using conventional detection technologies are described in the patent specification published under US 2007/0054326 (Ruo-Pan Huang, published on 8 March 2007).

The antigen array preferably comprises at least one antigen which is able to bind specifically to an antibody which is specific for a condition or disease to be tested for.

The antigen array or antigen microarray may have identical features.

According to the invention a series of capillaries, in other words an array of capillaries, i.e. a capillary channel system is used as an antigen array, these embodiments will be described in details further below.

According to the invention, on the antigen array such antigens are used which themselves do not cause cell adhesion, neither directly, nor through binding ligands recognised by the adhesion receptors of the detection cells from the sample, thereby ensuring that the detection cells indeed detect the antigen-specific antibodies and the immune complexes comprising same, respectively. Receptors responsible for adhesion of detection cells include e.g. integrins having various ligands such as connective tissue proteins.

Antigen arrays, antigen microarrays can be prepared in the desired form.

The term 'solid support', as used herein, refers to a well-known solid material to which various components used according to the invention can be physically attached, i.e. on which the components used according to the present invention can be immobilized. The term 'solid support', as used herein, refers to a non-liquid and non-gaseous substance. Immobilized components of the invention can be associated with a solid support by covalent bonds and/or via non-covalent attractive forces such as hydrogen bond interactions, hydrophobic attractive forces and ionic forces, for example.

Examples of such solid supports include, but are not limited to, membranes, sheets, plates, plastics, gels, sols, glass, ceramics and metals. For a general discussion of microarrays and suitable supports, see Shalon et al. [Genome Research 6: 639-645 (1996)], LeGendre [BioTechniques 9: 788-805 (1990)], U.S. Pat. No. 6,197,599 and U.S. Pat. No. 6,140,045.

In a preferred instance of the disclosure, the array is formed with a membrane made from polymeric, elastomeric or other suitable membrane material.

Particular features of membranes which are desirable to optimise the disclosure include the ability to bind large amounts of proteins, the ability to bind proteins keeping their denaturation on a minimum level and the inability to bind proteins that are not proteins of interest, when used as described herein.

In the disclosure an array of capillaries is used, an inner surface of the capillary channels (capillaries) or the constricting elements optionally present in the capillary channels are preferably covered by a coating or a reagent (e.g. PVDF, nitrocellulose, nylon, poly-L-lysine, silane, agarose or modified variants thereof) that increase the antigen binding capacity of the material of the capillary channel (e.g. glass or plastic). The present disclosure contemplates the use of any such material as known to those skilled in the art for use in Northern, Southern or Western blotting.

Further, another aspect is that the solid support may display at most minimal cell-binding and cell-activation properties on its own, only, thereby the detection cells will be bound to and activated by the complexes formed by the antigen and the components of the fluid tested on the features of the antigen array (which is preferably an antigen microarray), only, and that background signals have to be minimal.
The antigen is applied to the solid support in a way to ensure that its antigenicity is preserved and that the components (typically antibodies) of the sample, e.g. of a biological fluid, which are able to recognise the native form of the antigen also recognise it in the solid support-bound form. Antigens can be identified by their spatial location as features of a one- or two-dimensional array. The precise spatial positioning of the antigens are guaranteed by appropriate mechanical, robotic and/or information technology solutions. The relevant application and fixing techniques are known by those skilled in the art. For the preparation of the antigen array, which is preferably an antigen microarray, useful techniques include, but are not limited to, contact or liquid jet printers, liquid dispensing, piezoelectric devices and photolithography. Antigens may be bound through diffusion, adsorption, absorption, covalent chemical binding or affinity binding. In order to preserve the structure of the antigen, attachment to the solid surface is preferably performed under controlled temperature and moisture content. The arrangement and dimensions of the antigen arrays and the diameter of the antigen spots may be varied depending on the use and according to the needs.

The antigens and reference materials (e.g. controls) forming the antigen array are called the features of the antigen array, wherein the surface and area necessary for the structural forming of the array features are inclusive.

The term 'one or more antigens' used throughout the description and claims refers to one sort of or more sorts of (i.e. different) antigens. The term 'one or more reference substances' used throughout the description and claims refers to one sort of or more sorts of reference substances.

Each feature of the array comprises an antigen or a reference substance, which characterizes the particular feature of the array. In certain embodiments a given feature of the array comprises one sort of antigen or one sort of reference substance.

The 'antibody', as used herein, may be a polyclonal or monoclonal antibody. Typical examples of antibodies are the immunoglobulins. Further, the term 'antibody' encompasses intact immunoglobulin molecules, chimeric immunoglobulin molecules, or Fab or F(ab')₂ or scFv or single-domain antibody fragments. Such antibodies and antibody fragments can be produced by techniques well known by a skilled person, such as those described in Harlow and Lane [Antibodies: A Laboratory Manual, Cold Spring Harbor, N.Y. (1989)] and Kohler et al. [Nature 256: 495-97 (1975)] and U.S. Pat. Nos. 5,545,806, 5,569,825 and 5,625,126.

The antibodies used in the present invention may be of any isotype (of classes IgG, IgA, IgD, IgE and IgM and of subclasses IgG1, IgG2, IgG3, IgG4, IgA1, IgA2 in humans). The antibodies used in the present invention may be of any glycoform. Both the isotype and glycosylation status of antibodies are known to affect the recognition of antibodies by cells.

Antibodies produced in vertebrates, their isotypes and glycoforms are known by those skilled in the art, and are all included in the definition of the antibody according to the present disclosure.

The complement system is a network of dissolved and membrane-bound proteins. The activation of the complement system can occur through any pathways known in the field (classical, lectin and alternative pathway), and this results in the activation of successive elements of the system, accompanied by enzymatic cleavage steps. As a result of the activation, certain elements (C4b, C3b) covalently bind to macromolecules in the environment; they form molecular complexes with enzymatic activity (C3bBb, C4b2b, C3bBbC3b, C4b2b3b) or with pore forming capacity (C5b-9). The native forms of complement proteins do not have receptors; however, activation products and cleavage fragments have various receptors. These complement fragments facilitate the attachment of phagocytes to antigens and to antigen-carrying pathogens.

The complement system only works in the presence of bivalent cations. The functioning (i.e. working) of the complement system may be inhibited through binding the bivalent cations by chelating agents. Therefore, when treating the antigen array, which is preferably an antigen microarray, with a sample, chelators may be used to inhibit the functioning of the complement system while the binding of antibodies to antigens is maintained.

'Immune profiling', as used herein, refers to the determination of the immunoreactivity of an individual using an antigen array comprising more than one antigen. The term 'immunoreactivity', as defined herein, does not only include the reactivity of antibodies but also the reactivity of other recognition molecules and the activation of the complement system initiated by the antigens. It follows that immune profiling includes antibody profiling. The functioning of the complement system may be inhibited according to the above, thereby reducing the immune profile if necessary while antibodies are still able to bind to their targets, i.e. a specific antigen. Thus, according to the disclosure, by inhibiting the functioning of the complement system the immune profiling performed by cells may be restricted to antibody profiling, if desired. The determination of the immunoreactivity performed under these conditions according to the invention provides information about the capacity of antibodies to recognise antigens and to activate cells.

Conditions whereby an antigen/antibody complex can form as well as assays for the detection of the formation of an antigen/antibody complex and quantification of the detected protein are standard in the art. Such assays include, but are not limited to, Western blotting, immunoprecipitation, immunofluorescence, immunocytochemistry, immunohistochemistry, fluorescence-activated cell sorting (FACS), fluorescence *in situ* hybridization (FISH), immunomagnetic assays, ELISA, ELISPOT (Coligan, J. E., et al., eds. 1995. Current Protocols in Immunology. Wiley, New York.), agglutination assays, flocculation assays, cell panning, etc., as are well known to the person of skill in the art.

The term 'immune complex', as used herein, refers to a combination consisting of at least one antigen molecule and one antibody molecule kept together by non-covalent bonds. The term 'immune complexes' also includes more complex combinations of many antibodies binding to a single antigen, as well as structures comprising other immune recognition molecules [mannose-binding lectin (MBL), complement C1q, pentraxins] and various products of complement activation (C3b, iC3b, C3d, C4b, C4c, C4d, C5b-9).

The term 'qualitative analysis of the composition of the immune complex', as defined herein, refers to determining in the immune complexes the presence of the components binding to the antigens and reflecting immunoreactivity, that are preferably antibodies and complement components, preferably in terms of their biological activity. It is known to those skilled in the art that in a given organism, antibodies with different isotypes and glycosylation status may bind to a given antigen. Similarly, antigens are known to be bound by various components of the complement system partly via the above antibodies, depending on the pathway and progression status of the activation. The presence of biologically more active antibodies and complement components in the immune complexes formed on the antigen array results in the adhesion of higher number of cells.

The term 'semi-quantitative analysis of the composition of the immune complex', as defined herein, refers to the determination of the amount (i.e. quantity) of the components binding to the antigens and reflecting immunoreactivity, that are preferably antibodies and complement components, preferably in terms of their biological activity. It is known to those skilled in the art that in the case of a receptor-ligand interaction of a given affinity lower quantities of the ligand are less able to activate a cell through its receptor. An increase in the quantity of ligands, preferably the antibodies and/or complement fragments taking part in the formation of the immune complexes, will be accompanied by an increase in avidity and thereby stronger interactions between the antigen array and the cells, and will result in the adhesion of a higher number of cells.

The term 'binding', as used herein, includes the well-known antigen/antibody binding as well as other non-random associations between an antigen and an antibody. 'Specifically binding' or 'specific binding', as used herein, describes the behaviour of an antibody or molecule by which it does not substantially cross-react with any antigen other than the antigen, or antigens, specified. For instance, 'specific binding' of an antibody to a class of antigens having an epitope in common is included in the term.

### Sample

As used herein, the term 'sample' includes biological samples of various types and/or origins, such as blood and other liquid (i.e. fluid) samples of biological origin. The term 'biological sample' also includes any sample of biological origin. Typically, the sample contains at least one component which may be captured by the antigens arranged on the antigen array, such components include e.g. antibodies and complement components.

The term 'sample' may also refer to a clinical sample, as well as to whole blood, cell supernatants, cell lysates, serum, plasma, cerebrospinal fluid, cultured cells and other biological fluids, and any purified or enriched sample obtained therefrom. A sample may be derived from animals, e.g. mammals such as humans. These samples can be prepared by methods known in the art such as lysing, fractionation, purification, e.g. affinity purification, FACS, laser microdissection or preparative centrifugation.

As used here, the term 'whole blood' refers to blood which may contain an anti-coagulant or may be anti-coagulated blood. Concerning its source, the blood may be e.g. venous blood or, preferably, capillary blood taken from fingertip. Anticoagulants commonly used (EDTA, sodium citrate, heparin) affect the functioning of the complement system, which has to be taken into account when evaluating cell detection. Using a suitable anti-coagulant (e.g. hirudin) the coagulation system can be inhibited independently of the complement system. Preferably, such an anti-coagulant is used which does not influence the ability of the cells to adhere, or the influence is exerted in a planned manner.

The term 'capture protein', as used herein, refers to an immobilized protein which binds, or forms a complex with, one or more analytes of interest in the sample to be tested. Capture proteins are able to bind to their targets with high affinity, thereby capturing them from the tested sample, e.g. a biological fluid. Capture proteins may be used as features of the antigen microarray to provide controls for the measurement, to facilitate the evaluation of the results as reference substances. Antibodies are examples of capture proteins.

The term 'analyte', as used herein, refers to any molecule that contributes to the immunoreactivity of tested sample and binds specifically to a feature, or to multiple features, of the antigen array, which is preferably an antigen microarray. Analytes are preferably antibodies, most preferably antibodies of the IgG class, as well as complement components, preferably complement C3 and C4 fragments.

In the antigen array, the antigens themselves also act as capture proteins and bind at least one component, preferably an antibody, of the sample tested, thereby forming an immune complex.

### Reference substances

The term 'reference substances', as used herein, refers to the features of the antigen array which are used in the evaluation of the measurement rather than for the characterisation of the immunoreactivity of the sample. In terms of measurement technology, reference substances include positive controls, negative controls and standards.

Positive controls used for the cellular detection carried out on the antigen array guarantee cell adhesion in the analysis (testing) of any sample. Useful positive controls include proteins derived from connective tissues, preferably collagen; antibodies, preferably IgG molecules; capture proteins, preferably bacterial superantigens, as well as anti-human IgG antibodies; complement proteins, preferably vitronectin. In certain embodiments the positive controls are selected from the group consisting of superantigens, connective tissue proteins and materials other than antigens (non-antigenic materials), preferably antibodies and complement proteins.

In a preferred embodiment, a capture protein printed on the support of the antigen array, which is preferably an antigen microarray, as reference substance is an antibody specific for a component of the biological fluid to be tested. In a further preferred embodiment, a human IgG specific antibody is used as a capture protein, whereby the captured human IgG serves as a positive control for the adhesion and activation of the detection cells. In another preferred embodiment the adhesion of the cells is guaranteed by connective tissue proteins or suitable peptide segments thereof as positive controls.

Negative controls used in the cellular detection carried out on the antigen array do not enable cell adhesion. E.g. serum albumin may be used as negative control. In certain embodiments the negative controls are selected from the group consisting of albumins and materials other than antigens.

'Standards', as used herein, allow for the quantitative evaluation of the tests and for the compensation of the technical variability of the technology. Serial dilutions, i.e. solutions of appropriate dilution, of positive controls may be used as standards.

### Cells

'Cells', as used herein, refer to the basic units of life, in the biological sense. As the simplest biological units, cells are equipped with a machinery to decipher stimuli from the outside world and to respond to those stimuli. Current scientific knowledge and technology allow the modification of cells, these modifications range from the semi-synthetic forms of cells to genetic modifications, to metabolic modifications and to chemical modifications. All of such modified cells or cell forms are included in the scope of the present invention. Cells of the immune system, e.g. leukocytes or white blood cells, are particularly sensitive to complexes comprising immune recognition molecules, especially antibodies and complement components.

The term 'detection cells', as used herein, refers to cells which will be bound by the one or more immune complexes formed by the sample and the antigens, or which bind to, or form a complex with the immune complexes. Detection cells are preferably bone marrow-derived cells, e.g. monocytes, most preferably neutrophil granulocytes. Detection cells may carry a detectable, measurable label. Examples of measurable labels include, but are not limited to, nucleic acid labels, chemically reactive labels, fluorescence labels, enzymatic labels and radioactive labels.

Detection cells can be used also in unlabelled form, if the adhesion itself and/or its physical consequences are measurable. The adhesion of detection cells may have optical or electric effects or effects exerted on the flow of liquid (i.e. flow of fluid).

A combination of different sorts of cells and/or of cell fragments may be also used as detection cells, as an example the combination of monocytes, neutrophil granulocytes and thrombocytes in the blood can be mentioned.

The term 'cells' encompasses also those cells and cell fragments of the sample itself, which are suitable for implementing the method of the invention. As whole cells neutrophil granulocytes, as cell fragments platelets can be given as examples. In a preferred embodiment, cell lines with appropriate receptors are used for detection. Human immortal monocytoid cells expressing various immunoglobulin Fc and complement receptors are well known to those skilled in the art (for example, U937, THP1, MonoMac). In another preferred embodiment, immortal cells are transfected with receptors specific for immunoglobulins and complement components, thereby rendering them suitable for detecting immune complexes on antigen arrays, which are preferably microarrays. In another preferred embodiment, primary human monocytes are used as detection cells. The array-based detection provides information about the function of the monocytes and this reflects the immune status of the blood donor individual.

A 'receptor', as used herein, refers to a protein which is embedded in the plasma membrane or in the cytoplasm of a cell and is able to bind a 'ligand', i.e. a specific molecule that fits into the binding pocket of the receptor. Each cell typically has many receptors of different kinds, with different ligand-binding abilities. Binding of the ligand induces a change in the state of the receptor regarding its conformation or oligomerisation, and also perturbs the signaling molecules attached to the receptor. Cells will integrate various signals from the different receptors and certain cellular responses will follow.

In another preferred embodiment, genetically engineered cell lines can be used for detection in a method of the present invention or in the kits according to the disclosure. In case cells are genetically engineered to allow the monitoring of cell activation, or in case an assay detecting cell activation is used, cellular detection over the one by detection antibody is even more preferable. Thus, concrete evidence is provided as to whether the immune complexes formed can activate the cells in case the antigen enters the body. In addition to inducing inflammation, cell activation may have a very wide range of other effects in the body and these may be important information.

In a preferred embodiment, detection cells are fluorescently labelled to allow the measurement using fluorescence microarray scanners. Methods for obtaining fluorescent signals in cells are known to those skilled in the art. Both constitutive and inducible fluorescence can be achieved by chemical and/or genetic modification of the cells. These technologies include the use of cell permeable dyes (CFSE, Cytotracker®), dyes sensitive to oxygen radical species (Rhodamin 123), and dyes that accumulate in various intracellular compartments or in lipid layers. Genetic modification may include the introduction of genes encoding fluorescent proteins or fusion proteins with fluorescent fusion partners (GFP, YFP, etc.) and these may be constitutively expressed or may be under the control of transcription factors that reflect cellular activation state. As an alternative to labelling the cells prior to the application to antigen arrays, preferably to antigen microarrays, intracellular components, surface markers or cell products may also be labelled posteriorly, that is, when cells had adhered to, been activated by or responded to the complexes on the antigen array, which is preferably an antigen microarray.

Detection cells may derive directly from the donor of the biological sample, that is, in this embodiment both the biological sample and the detection cells are derived from the same subject, e.g. individual. An outstanding advantage of such a system is that direct information can be obtained as to what happens when the antigen is contacted with the serum of the donor (e.g. a patient) and with the donor's own cells, thereby approximating the processes actually occurring in the body ('personalized medicine').

In human blood the white blood cell of highest number is neutrophil granulocyte, in healthy adults the number of neutrophil granulocytes is 1 500 - 8 000 per microliters. These cells are responsible for destroying pathogens, however, in autoimmune processes they may attack also the self organism. In these processes the interactions between the Fc receptors and the target-bound IgG antibodies play an important role. On the basis of the above, neutrophil granulocytes present in whole human blood are suitable as detection cells on the antigen array. In an instance of the disclosure, it is utilised that the activation of neutrophil granulocytes may lead to the discharge of nuclear content and antimicrobial peptides. The phenomenon is called NETosis deriving from the English term 'Neutrophil Extracellular Traps' (Brinkmann, V. et al. 2004. Neutrophil extracellular traps kill bacteria. Science. 303:1532-1535.). The further activation of the already activated and immunocomplex-bound neutrophil granulocytes may lead to the induction of NETosis. The discharge of the nucleus content leads to the formation of chromatine network (NET): the chromatin present in the nucleus in a compact form occupies a much larger space outside the cell in an unfolded form. The extracellular chromatine can be detected through DNA dyeing, or, without labelling, through slowing down of the liquid flow.

In a preferred embodiment whole blood is used on antigen arrays formed of capillaries. Preferably blood obtainable from fingertip is used, which has the advantages that no medical qualification is needed for taking the sample and that the blood obtained from fingertip contains a higher number of neutrophil granulocytes compared to the venous blood. Preferably detection is carried out without labelling, on a capillary array device suitable for rapid diagnostic method.

So, in the methods of the invention, the cells can act as detection cells (sensors) that bind to complexes formed by selected antigens and the components (typically molecules) of the bodily fluid or solution. Contacting the antigen array with bodily fluid or solution comprising said molecules allows the binding of certain immune recognition molecules, preferably antibodies, to the antigens. The detection indicates the presence of molecules responsible for immunoreactivity in the bodily fluid or solution, which molecules act as recognition molecules for certain antigens, and also indicates the fact that cells recognise these antigen-bound molecules by their receptors, the receptors promote the adhesion and activation of these cells. Preferably, such components are antibodies and/or complement fragments. Preferably, the antigens are autoantigens and/or pathogen-derived antigens.
In the methods of the present invention, when the antigen array is contacted with a sample, the formation of complexes between the antigens and antigen recognition molecules can be achieved under a variety of conditions. Typically physiological pH, salt concentrations and temperatures are used. It is well known that by adjusting the conditions of this contacting step, which optionally comprises also an incubation step, components can be deliberately controlled to bind or not to bind. Dilution of the sample favours the binding of high affinity agents. Conditions that favour the activation of the complement system will result in the deposition of complement components that will have a serious impact on the cellular recognition of the complexes thus formed. The present invention incorporates all kinds of conditions as long as they do not inhibit cell binding (adherence) to components on the antigen array. Typically physiological pH, salt concentrations and temperatures are used.
With the current protein array technologies, macromolecular interactions can be monitored by both label-dependent and label-free methods. Label-dependent methods use affinity reagents, e.g. antibodies, labelled appropriately to allow their binding to particular surfaces of the protein array. Label-free methods sense and quantify physical events induced by macromolecular interactions. Both approaches interpret macromolecular interactions as biophysical-biochemical events and the experimenter extrapolates to potential biological events that could occur under *in vivo* conditions.
In a further step towards modelling biological functions on arrays, the inventors have applied cells to the arrays with the purpose of probing the printed array content. In the course of probing by cells macromolecular interactions take place between a printed array feature and a cellular component. The present disclosure takes one further step by using cells for the analysis of interactions between a printed microarray feature, or features, and a component, or components, of a tested sample, that is, to assess whether any interaction takes place, and if so, with what intensity, assuming that complexes formed by the macromolecular interactions further interact with the components of the detection cell in a way that characterizes the former complex.

In the methods of the present invention, preferably those components of the tested sample will interact with the antigens which are antibodies and complement components, and the receptors of the detection cells will bind to said sample components. Antibodies bind to those features of the antigen microarray towards which the tested sample shows immunoreactivity. The quantity and quality of bound antibodies characterise the immunoreactivity of the organism providing the sample. The quantity and quality of bound antibodies determine the quality and intensity of complement activation.

The components of the sample which may be captured by the antigens, typically antibodies and complement components, bind to the features of the antigen array, which is preferably an antigen microarray, and form immune complexes on the surface of the solid support. The receptors of the cells used for the detection (Ig Fc receptors and complement receptors) interact with them with an intensity depending on the composition of the immune complexes, mediate signals into the cells and facilitate their adhesion to the immune complexes on the surface of the antigen array, which is preferably an antigen microarray. The adhesion and activation of the detection cells will characterise the quality of the immune complexes, and the efficiency of the adhesion may be measured by monitoring the number and activation status of the adhered cells. Antibodies efficiently recognising a given feature of the antigen array, which is preferably an antigen microarray, promote an increase in the number of cells adhering to the area of a given feature of the array.

It is generally known that cells of the immune system constantly monitor the body and are equipped with receptors suitable for recognizing both non-self molecules posing danger for the organism itself (e.g., TLRs, NLRs, CLRs, scavenger receptors, etc.), as well as certain self molecules (e.g., integrins, FcRs, CRs, etc.). Immune recognition molecules are also present in the serum in soluble forms, and these include, for example, pentraxins, MBL (mannose-binding lectin), LBP (lipopolysaccharide-binding protein), etc. Of note, antibodies are also soluble receptors of the B lymphocytes. It is also commonly known that the binding of soluble recognition molecules to antigens will enhance the recognition of that antigen by the concerted action of cell surface receptors.

### Detection - evaluation

The analysis of the cells adhered on the antigen arrays or of the immune complexes may be quantitative, semi-quantitative or qualitative. 'Detection by cells' or 'detecting by cells' refers to determining which tested antigens formed complexes, and, optionally, also the amounts (i.e. quantities) of the complexes formed, and/or to identifying those tested antigens that are not involved in complex formation on the antigen array (i.e. ascertaining lack of complexes), which array is preferably an antigen microarray. 'Lack' of binding or complex formation, or 'lack of detection of product', as used herein, means insignificant levels. Detection by cells typically comprises revealing adhered (bound) cells, determining the number/amount of adhered cells, which indicates the extent of cell adhesion, and/or measuring the activation of adhered (bound) cells, or may consists of one or more of these. Measurement may refer to sensing, and/or measuring the extent to which cells adhere and/or become activated, measuring the amount of adhered cells, etc. Revealing the cells adhered on the areas of the individual features of the antigen array comprises identifying those tested antigens on the area of which cells adhered, and, optionally, also determining the amounts of the adhered cells, and/or comprises identifying those tested antigens on the area of which no cells adhered.

Detection or revealing will be typically carried out also for the reference substances.

According to certain instances of the disclosure, the cells are detectably labelled cells and the step of detecting by cells comprises measuring the amount of the cells that adhered to the immune complexes formed by the components of the sample and the antigens of the antigen array, or measuring a physicochemical attribute proportional to said amount.

According to certain instances of the disclosure, the detecting by cells, the step of revealing the adhered cells, the determining the number of cells adhered and/or the measurement of the activation of adhered cells comprises measuring cellular response, wherein the cellular response preferably is selected from: production of reactive intermediers, increase in intracellular ion concentrations, phosphorylation of signaling molecules, and altered expression of surface markers, all induced by the contact between detection cells and immune complexes formed by components of the sample and antigens of the antigen array.

According to certain instances of the disclosure, the detecting by cells, the step of revealing the adhered cells, the determination of the number of cells adhered and/or the measurement of the activation of adhered cells comprises measuring cellular responses using genetically engineered cells, chemically modified cells, primary cells isolated from a donor or immortalised cell lines.

According to certain instances of the disclosure, the step of detecting by cells, the step of revealing the adhered cells, the determining the number of cells adhered and/or the measurement of the activation of adhered cells comprises a labelling-free measurement of cell adhesion or cell activation through exploiting physical, optical, electrical, mass or magnetic alterations induced by the adhesion or activation of the detection cell.

Depending on the application, the contacting time may range from 1 second to 1 day, but typical contacting times are 10 to 60 minutes. The temperature used for the contacting step ranges between the freezing point of water and the upper temperature value which does not cause damage to cells, and is typically about 37°C.

Buffers used in the method have a close-to-neutral pH (6 to 9), physiological ion strength and osmolarity. The physiological functioning and viability of the cells are ensured by the liquid media used in the method.

Cell numbers required for the uses according to the present invention are as follows:
- in relation to the antigens, at least 1 cell per antigen, preferably a number of cells able to cover entire antigen spots (the entire surface of the antigen feature), for example, at least 2,000 cells per antigen if antigen spots of a diameter of 500 micrometers are used;
- in relation to the antigen array, which is preferably an antigen microarray, at least 1 cell for each feature of the antigen array (preferably an antigen microarray), preferably a number of cells able to completely cover each feature of the antigen array (preferably an antigen microarray), more preferably a number of cells able to cover the entire surface of the array, for example, 400,000 cells per 0.3 cm².

Preferably, the antigen array is not shaken.

The sample may be used in a concentrated form or in a diluted form, e.g. in a 5-fold dilution, in order to approximate natural conditions as much as possible. Preferably, the 'contacting' according to the present disclosure comprises also incubation.

It is not necessary to simultaneously contact all features of the antigen array with the sample and/or with the cells.

In a preferred embodiment, a washing step is performed after contacting the antigen array with the biological sample and/or cells not able to interact with the complexes formed on the array, which is preferably an antigen microarray, are removed by washing. Various solutions (for example, phosphate-buffered saline) may be used for removing unbound (non-adhered) cells, as long as cellular integrity in maintained. Once unreacted detection cells have been removed, array slides may be archived by various methods, such as fixation or drying. For archiving such a method has to be selected wherein the signals localized to the array features are preserved.

The reaction can be considered as positive, if higher number of cells adhere on the area of the antigen tested (e.g. antigen spot) than on the area of the negative control antigen/substance, or, on the test surface not covered by antigen (background adhesion), respectively. If the number of cells adhering on the area of the antigen tested or the number of cells activated thereby is not higher than the numbers for the area of the negative control, and the positive control indeed worked, the reaction is considered as negative. Positive control can be considered as working if on its area the number of adhered cells or the number of activated cells is higher than on the area of the negative control substance. The reaction cannot be considered as negative if the number of cells adhered to the positive control is not higher than either that on the negative control antigen/substance, or on the test surface not covered by antigen (background adhesion).

### Further description of capillary channels, capillary channel systems, microfluidic devices, and methods implemented through their application

An array of capillary channels i.e. a capillary channel system is used as an antigen array.

The invention encompasses a method for analysing immunological reactivity, as defined in the claims, comprising introducing a sample into a capillary channel system according to the invention, and observing or measuring in the reader section in each channel a flow characteristic, preferably the flow rate or a proportional quantity once a liquid front of the sample has reached the reader in the negative control channel, preferably once a liquid front of the sample has reached the reader in the negative control channel as well as in the measuring channel.

The sample is preferably blood.

Unless indicated otherwise, the description above also applies to the implementation of the methods according to the invention in the capillary channel systems.

According to the above, the invention also relates to a capillary channel as defined in the claims, preferably a microfluidic system, these are especially suitable for implementing the methods according to the invention.

The measuring channel comprises a trap (also called 'a trap section') haying an antigen coating, further comprises a collector section (also called 'a collector' or 'a collector container section') having a volume which is at least as large as that of the trap section, and a reader section (also called 'a reader') adjacent to the collector section, which reader section optionally comprises one or more physical and/or chemical sensors adapted for measuring a liquid flow rate and/or it comprises one or more markings for visual reading of a liquid front position, and wherein the collector section is located in the capillary channel between the trap section and the reader section.

Hence, the reader section is adjacent to the collector section. The adjacency of the two sections may also mean according to the invention that the reader section is inside the collector section, in such a part thereof, which is farther from the trap at least with a distance corresponding to the volume of the trap section. When there is liquid in the reader, the flow rate of the liquid optionally visible to the naked eye can be observed, read or measured in the reader. So as to enable reading, the reader section preferably has a transparent wall. Liquid can be introduced into the capillary channel through its inlet aperture.

The capillary channel system according to the invention comprises at least one measuring channel, which measuring channel is a capillary channel, in which the coating comprises an antigen, and the system comprises at least one negative control channel, and furthermore optionally at least one positive control channel, and optionally at least one sample distributing part, which communicates with the channels.

The negative control channel is preferably such a capillary channel which has a structural configuration identical with that of at least one measuring channel, and it Is provided with a coating other than that of the measuring channel or it is formed without coating. The positive control channel is preferably such a capillary channel which has a structural configuration identical with that of at least one measuring channel, and it is provided with a coating other than that of the measuring channel and negative control channel of identical structural configurations.

In the capillary channel system, the one or more measuring channels may have identical or different structural configurations. The coatings in the trap section of the different measuring channels may be identical or different. Typically, separate control channels (positive and optionally negative) are provided for the measuring channels of different structural configuration. If there are measuring channels of different structural configuration in the capillary channel system, it is preferred to use at least one negative control channel corresponding to each structural configuration, and optionally at least one positive control channel corresponding to each structural configuration in the system. The control channel of a configuration identical with that of a given measuring channel is also called a measuring channel associated control.

The capillary channel system according to the invention may be a microfluidic device/apparatus or a part thereof.

Phenomena and rules governing liquid flow in the micrometer range are known to a person skilled in the art, and they are described by the science of microfluidics.

The sections (trap, collector, reader), represent a communicating channel system, and the dimensions and inner volumes of the sections may change subject to the type of use of the capillary channel.

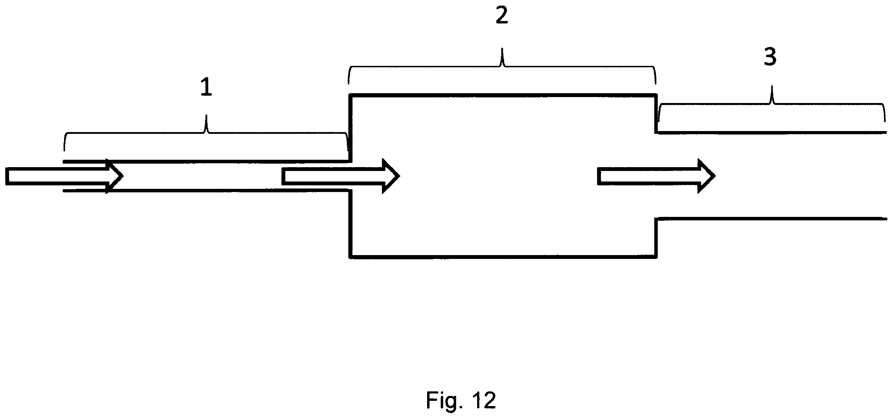

Fig. 12 shows an embodiment of the capillary channel of the capillary channel system, which in the present embodiment comprises a trap section 1, a collector section 2, and a reader section 3. In Fig. 12, the arrow shows the flow direction of the sample in the capillary channel, if the capillary channel is used in the method according to the invention.

In a more simple embodiment than that shown in Fig. 12, the capillary channel is a capillary tube, and the collector is located in the same capillary tube with the trap and the reader (the collector and the reader are only separated functionally and not physically). In this case the reader can be defined as a capillary tube section downstream from the collector having the same volume as the trap. If the collector and the reader are not separated physically, the starting point of the reader may be shown by a marking on the capillary channel.

A capillary channel may comprise more than three sections.

In the capillaries, a liquid may flow subject to its interactions with the capillary wall. If the liquid wets and adheres to the capillary wall, then the liquid is moved along, i.e. it is 'taken into' the capillary channel by the capillary forces. Accordingly, if desired, a liquid e.g. a biological sample is introduced into the capillary channel, e.g. into a capillary tube in accordance with the principle of capillarity. This means that if a capillary channel, e.g. a capillary tube is e.g. immersed into liquid, the sample enters into the capillary channel in accordance with the principle of capillarity. If the capillary channel is not sufficiently hydrophilic to ensure that the sample flows in the channel on the basis of the capillarity principle, external intervention (e.g. centrifuging of the device after the introduction of the sample or moving the sample in the device by a suction or pressure force) may be required for forcing the sample flow through the device (capillary channel, capillary channel system).

Preferably, the wall of the capillary channel is made of glass and/or polymer, and the inner hydrophilic surface assists capillary forces in moving aqueous liquid. The walls of the various sections of the capillary channel may also be made from different materials. When the capillary channel of the capillary channel system according to the invention is designed to allow a biological sample to flow through it, e.g. for the implementation of a method according to the invention, the material of the capillary channel is preferably a material or several materials that do not enter into biochemical interaction with the sample to be analysed.

The capillary channel may be, for example, a simple capillary tube, any cross-section of which is of the same size, or may be a complex capillary tube which consists of several simple capillary tubes, in which any cross-section of each simple capillary tube is of the same size, or may be a simple or complex capillary tube, the trap of which comprises a constriction as a result of applying one or more constricting elements or using a trap wall designed to have a constricting effect.

### Trap

The important structural characteristics of the trap are the length, the shape, the cross-sectional view and the cross-sectional area traversable to (i.e. passable for) the liquid, and that whether the traversable cross-sectional area is constant in the full length of the trap section. In an embodiment of the capillary channel of the capillary channel system according to the invention, the trap section is formed with at least one route (a passage which ensures passing between the beginning and end of the trap section) which has a traversable cross-section having a characteristic size of 1 to 50 micrometer.

The material of the trap wall determines the extent of liquid capillary forces, and the options of coating the inner surface and linking the biological molecules. The coating of the trap is typically applied to a ground layer. The most important biological and biochemical characteristics of the trap are the material of the ground layer, and the quality of the coating, e.g. antigen coating (i.e. the applied antigen), and antigen density on the surface ground layer. In certain embodiments, there is no coating in the trap of the negative control capillary channel.

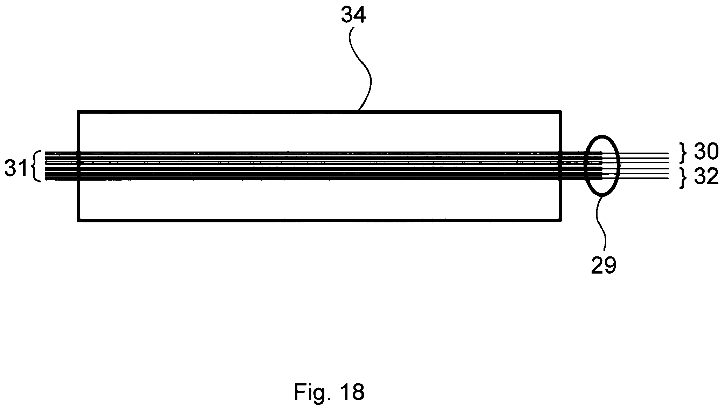

In the trap section, the inner surface of the capillary channel wall (or in certain embodiments that of a route which ensures transfer between the beginning and end of the trap section) and/or the outer surface of one or more constricting elements located in the trap carry the coating, i.e. is (are) coated with a material, e.g. antigen (cf. the embodiment in Fig. 18).
For instance microbeads, fibres, a capillary of a diameter smaller than that of the capillary channel, etc. can be used as constricting elements. The above mentioned inner or outer surface may be coated partly or fully with a material, e.g. antigen.

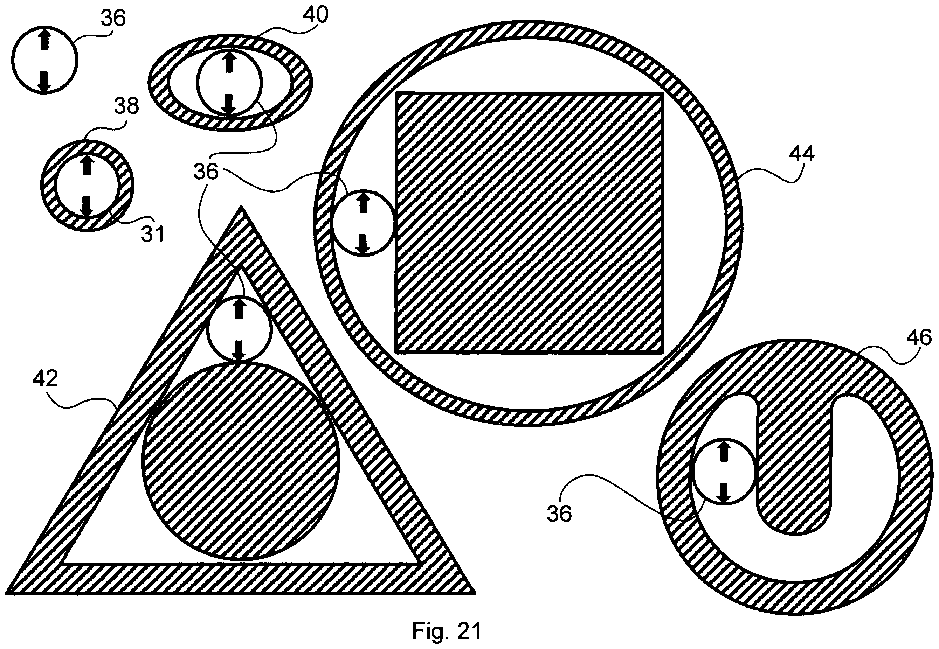

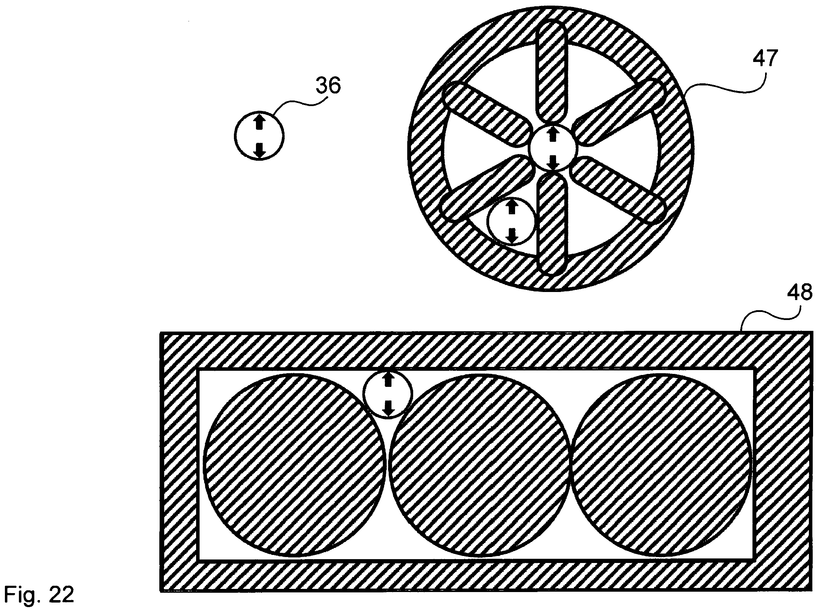

In embodiments shown in Figs. 21 and 22, the trap section comprises at least one constricting element, and the traversable cross-section of at least one route is formed with at least one constricting element. The constricting element may have a varying size and/or shape along the length of the trap.

The at least one passage in the trap section has a traversable cross-section, which - as shown in Figs. 21 and 22 - may even have an irregular shape, especially in case the passage is formed with constricting elements in the measuring channel. A characteristic size is assigned to the traversable cross-section, which specifies the linear size in which the section of maximum length can be inserted into the traversable cross-section. Since the shape of the objects going through the passage is close to spherical in many cases, the characteristic size of the traversable cross-section may also be defined by the diameter of the largest circle that can be inserted into the traversable cross-section. Such a definition of the characteristic size is illustrated in Figs. 21 and 22.

Thus, the characteristic size of the traversable cross-section specifies the approximate characteristic size of the object which can still pass through the traversable cross-section. Here it is to be taken into consideration that both the traversable cross-section and the objects passing through them have irregular shapes in many cases.

When the capillary channel of the capillary channel system according to the invention is used in the implementation of a method according to the invention, in a preferred embodiment the coating of the trap section of the measuring channel comprises an antigen which measures a given reactivity. A trap comprising an antigen enables the adhesion of cells of a sample, preferably neutrophil granulocytes present in the blood, maybe monocytes and thrombocytes, in this section to the immune complexes formed by the antigen present on the inner surface (optionally on ground layer) of the capillary channel - in certain embodiments the passage - wall or on the outer surface of one or more constricting elements in the trap (optionally on ground layer) and by the antibodies present in the sample flowing through, in such a way that the adhesion leads to a slow-down of the liquid flow as a result of reducing the traversable cross-section. By appropriately configuring the structure of the trap, the efficiency of cell adhesion, the resulting changes in the liquid flow and hence the sensitivity of measurement are regulated. Resulting from the dimensions of the trap, the processes taking place in the trap section are determined by the rules of microfluidics known to persons skilled in the art. The adhesion of cells may be facilitated e.g. by extending the length of the trap, forming the shape and the cross-sectional view so as to enhance turbulence, and reducing the traversable cross-sectional area.

The adherent cells in the blood have a diameter smaller than 20 micrometers, and, accordingly, when creating a traversable cross-sectional area, preferably inner distances shorter than 20 micrometer, and even more preferably shorter than 10 micrometers have to be formed.

The neutrophil granulocytes in the blood are able to change their shape substantially, and they are even able to pass through pores of 1 micrometer diameter, therefore the smallest diameter in the cross-sectional area may even be as small as 1 micrometer, if necessary. Excessively facilitating cell adhesion may not only lead to increasing the sensitivity, but also to extending the measuring time. In the trap section, the characteristic size of the traversable cross-section in an embodiment of the capillary channel of the capillary channel system according to the disclosure is between 1 and 50 micrometers. The lower limit of the characteristic size is determined by the fact that preferably most of the objects frequently emerging in the sample, especially those significant from the aspect of the invention, must be able to pass through. Therefore, the characteristic size shall be at least 1 micrometer, because a narrower passage would not function. The characteristic size can be advisably larger so that it most probably does not represent an obstacle to any component of the sample to be analysed.

The characteristic size may be up to 50 micrometers, i.e. it is 50 micrometers or less, because in the case of using as broad passage as possible, it may happen with an increasing probability that adhesions to the channel wall are unable to have an effect that changes the flow characteristics of the sample going through the passage. The characteristic size is preferably less than 50 micrometers so that a change in the flow characteristics of the sample may be observed already in the case of a relatively smaller adhesion rate.

In our experiments we have found that due to the phenomena described above, it is advisable to form the trap section with a passage having a characteristic size of 5 to 20 micrometers. With this characteristic size, the impacts experienced optionally in the vicinity of the lower or upper boundary of the characteristic size may be preferably avoided, and furthermore in the case of applying these characteristic sizes, the capillary channel of the capillary channel system according to the invention works appropriately within a broad range of samples.

As illustrated in Figs. 21, 22 and 28, several passages may be located in the trap section. It is necessary to apply the characteristic sizes specified above to each of these passages to ensure that the capillary channel of the capillary channel system according to the invention works appropriately. The working of the capillary channel of the capillary channel system according to the invention would be detrimentally influenced, if even one of the characteristic sizes assignable to several passages were larger than 50 micrometers. The characteristic sizes of the passages located in the trap section are preferably identical. It is advisable to apply identical characteristic sizes, because in this case the flowing sample will be distributed evenly in the passages, and, therefore, due to the adhesion the flow characteristics will change roughly equally in each passage.

In certain preferred embodiments, in any cross-section of the trap, the traversable cross-sectional area, i.e. the characteristic size of the cross section of the route (i.e. that part of the cross-section which is accessible to a liquid flow) at any place may be up to preferably 20 micrometers, and especially preferably 10 micrometers or less, and it is permeable to regular spherical beads.

Such a sphere 36 is illustrated in Fig. 21 on its own and in trap sections of various cross-sections. In a trap section 38 of circular cross-section this means that the inner diameter (ID) of the capillary is preferably 20 micrometers or less, and especially preferably 10 micrometers or less. In a trap section 40 of oval or elliptical cross-section, the distance between the closer opposite walls or the inner minor axis of the capillary is preferably 20 micrometers or less, and especially preferably 10 micrometers or less. In capillaries of a circular or other geometrically shaped cross-section comprising a constriction as a result of the configuration of the wall or due to inner constricting elements, the largest diameter of a circle that can be inserted into the traversable cross-sectional areas is preferably 20 micrometers. Such further trap sections 42, 44, 46, 47 and 48 are illustrated in Figs. 21 and 22.

The wall of the trap section 42 surrounds a triangular cross-section inner space, in which an inner constricting element of circular cross-section is arranged. The trap section 44 is formed in a way that in the inner space of the cylindrical capillary, a constricting element of square cross-section is positioned, and the largest distance between the constricting element and the capillary channel wall is preferably 20 micrometer, and especially preferably 10 micrometers.

The trap section 46 has a cross section of circular outer periphery, and a protrusion extends into its inner space in a way shown in the figure, so that - preferably along a certain length of the capillary - the largest size of the traversable cross-section is reduced preferably to 20 micrometers and especially preferably to 10 micrometers.

Fig. 22 illustrates two further capillary cross-sections. In the trap section 46, the protrusions extending towards the centre of the capillary are arranged like spokes in a way that the largest size of the traversable cross-section is preferably 20 micrometers and especially preferably 10 micrometers. The trap section 48 has an inner space of rectangular cross-section, in which inner constricting elements of circular cross-section are arranged in a way that in the inner space of the capillary channel, the largest size of the traversable cross-section is preferably 20 micrometers, especially preferably 10 micrometers.

Preferably the trap is a section of 0.1 to 20 mm length.

The solid support, its composition determines which coating, e.g. antigens, can be bound to the surface and how. The materials, reagents and techniques adapted for applying (binding) various materials, e.g. antigens, are known to a person skilled in the art. In the case of a trap made of glass, for example, poly-L-lysine, silane, nitrocellulose and agarose are suitable for binding protein materials, e.g. protein antigens. The ground layer may comprise a material which improves cell adhesion triggered by antibodies. Such materials are for example fibronectin and vitronectin. As the density of the antigens determines the highest possible density of binding antibodies, the same contributes to the elaboration of the measurement sensitivity. By using ground layer forming (surface coating) materials of higher capacity and antigens of higher concentration, the sensitivity of the measurement can be increased. The adhesion of cells, through prior binding of antibodies from the sample, may be achieved preferably by the use of antigens of 0.1 to 10 mg/ml application concentration. The antigen density achieved can be determined in the trap by techniques known to a person skilled in the art, for example through the use of enzymatically labelled antigen specific antibodies, by measuring the bound enzyme activity.

The biological-biochemical characteristics of the trap also regulate the sensitivity of the measurement.

Collector - When the capillary channel or capillary channel system according to the invention is used in a method according to the invention, the role of the collector in the measuring channel comprising the antigen is to ensure the receiving of a sample in such a volume, which, at the time of entering the capillary channel, comprises an antibody quantity sufficient to trigger cell adhesion after the binding of the antibodies to the antigens of the trap. The collector plays a passive role during the methods according to the invention, but by selecting its volume, the sensitivity can be regulated: by increasing the volume, a larger quantity of sample can flow through the trap, and antibodies of lower concentration may also be bound there in a sufficient quantity, thereby increasing the sensitivity of the measurement.

Reader - Optionally, in the reader section, the rate of a liquid flow can be visually monitored and/or measured. Preferably, the wall of the reader is transparent, which enables the tracking and observation of the liquid flow.

In a further preferred embodiment, the reader comprises one or more physical and/or chemical sensors adapted for the quantitative measurement of the rate of a liquid flow, and this sensor or these sensors provide(s) electronic signals in case an instrument is connected.

Thus, the reader section preferably comprises one or more physical and/or chemical sensors and/or one or more markings which enable the visual reading of the position of the liquid front.

Optionally several traps with the same coating, e.g. antigen-containing coating, may be coupled to one collector and through this to a reader. In the case of several traps, the volume of the collector is at least as large as the combined volume of the traps.

In the capillary channel system, preferably the trap section of each capillary channel comprises one type of characteristic antigen or reference material.

The capillary channel system comprises at least one negative control capillary (also called as 'a negative control channel') and optionally also at least one positive control capillary (also called as 'a positive control channel').

The positive control channel is a capillary channel, the coating of the trap section of which comprises a positive control material. The negative control channel may be a capillary channel the trap section of which is coated, and optionally it comprises a negative control material, or it may be a capillary channel having preferably the same structural configuration as that of the measuring channel, but having no coating.

Two channels have the same structural configuration, if the structures of the individual channels (the length, width and cross-section of the corresponding sections, the constricting elements optionally arranged in the trap section) are identical, or symmetrical projections of each other, i.e. the channels of identical structural configuration only differ in the coating applied in their trap sections.

The capillary channel system comprises a measuring channel and a negative control channel, which have identical structural configurations. Preferably, the capillary channel system comprises such a negative control channel which - when an identical sample is flown through the measuring channel and the negative control channel - provides a flow rate identical with or higher than that in the measuring channel in the part downstream from the trap section. In this embodiment, the associated channels of preferably identical structural configuration are different in their coatings or the negative control channel has no coating, and when identical sample is flown in both the measuring channel and the negative control channel, and the corresponding points of the part downstream from the trap section of the measuring channel and the part downstream from the trap section of the negative control channel are compared, the flow rate of the liquid front of the sample in the negative control channel will be identical with or higher than in the measuring channel, because the trap section of the negative control capillary has no coating or its coating typically does not trigger cell adhesion in any sample to be tested. More specifically, if the sample comprises at least one component which can be captured by the antigen present in the trap section of the measuring channel, which said at least one component is selected from immunologically reactive components, preferably antibodies and complementary components, then in the negative control channel in the part downstream from the trap section the liquid flow will be faster than in the corresponding part of the measuring channel, provided that the measuring channel and the negative control channel are of identical structural configuration.

A preferred capillary channel system comprises a measuring channel and a positive control channel, which have identical structural configurations. Preferably, the capillary channel system comprises a positive control channel which - when an identical sample is flown through the measuring channel and the positive control channel - provides a flow rate identical with or lower than that in the measuring channel in the part downstream from the trap section. In this embodiment, the associated channels of preferably identical structural configuration are different in their coatings, and when the same sample is flown in both the measuring channel and the positive control channel, and the corresponding points of the part downstream from the trap section of the measuring channel and the part downstream from the trap section of the positive control channel are compared, the flow rate of the liquid front of the sample in the positive control channel will be identical with or lower than in the measuring channel, because the trap section of the positive control capillary has a coating which is expected to lead to a strong reaction, i.e. cell adhesion in the case of all samples to be tested.

In the case of the positive control channel such a situation may arise that the liquid front of the sample does not even reach the very point downstream from the trap section where the flow characteristics in the positive control channel, the negative control channel and the measuring channel are compared. Therefore, a flow rate slower than that in the measuring channel, i.e. a lower rate, encompasses also a meaning that the sample does not even reach the relevant point in the positive control channel, i.e. its rate is zero at the given point.

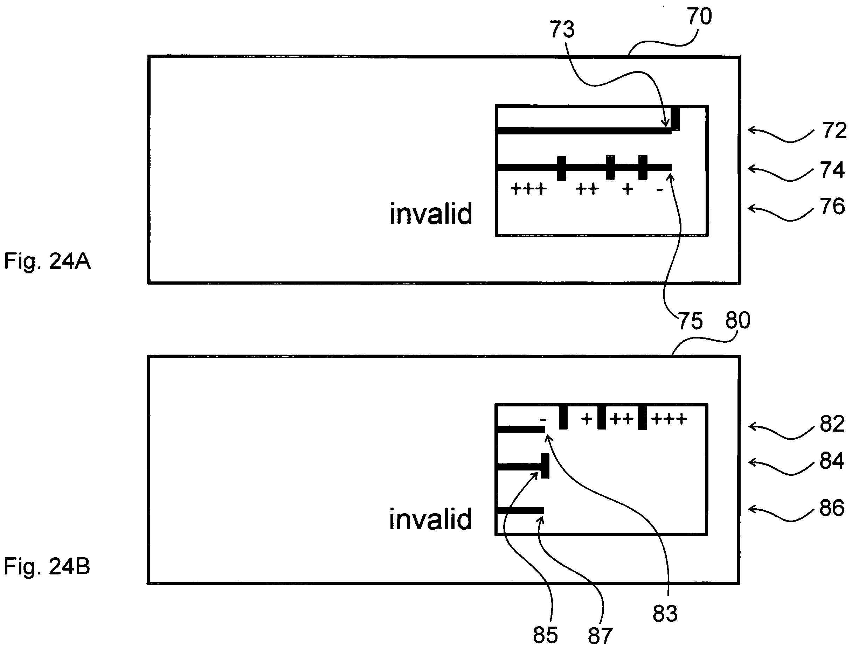

Accordingly, the flow in the section of the positive control channel downstream from the trap section is preferably slower and the flow in the section of the negative control channel downstream from the trap section is preferably faster than in the measuring channel associated with them. In the case of samples from which no substantial cell adhesion is revealed, it may happen that in the positive control channel, in the negative control channel and in the measuring channel identical flow rates are found. Such an example is shown in Fig. 24B.

In the most preferred embodiment of the capillary channel system (linear series or array of capillaries), as a result of the structure of the capillaries, the adhesion of the detection cells influences the rate of the liquid flow in the capillary. An optimal structure can be achieved by the appropriate selection of the internal diameter of the capillary, in addition to changing and regulating same in a direction perpendicular to the liquid flow.

Since the methods according to the invention are implemented through the use of the capillary channel or capillary channel system (microfluidic system) according to the invention, typically the extent of cell adhesion and the quantity of adhered cells are concluded from observing or measuring a flow characteristic of the sample. In order to obtain information about the quantity of adhered cells, a flow characteristic of the sample, e.g. its flow rate or a quantity proportional thereto is observed and/or measured. Observing the flow rate typically comprises that a reading is taken of the distance covered by the liquid front of the sample in a given time period. Measuring the flow rate comprises for example the measuring of conductivity or optical density. Cell adhesion from the sample causes a change in the flow rate of the sample. In a different case, observing and/or measuring a flow characteristic comprises that the stopping of the flow of the sample is detected.

Preferably, the capillary channel system comprises more than one capillary channel comprising a specific antigen or reference material, which enables performing parallel (simultaneous) measurements; i.e. in the capillary channel system one given type of antigen or reference material is comprised optionally in one or more capillary channels.

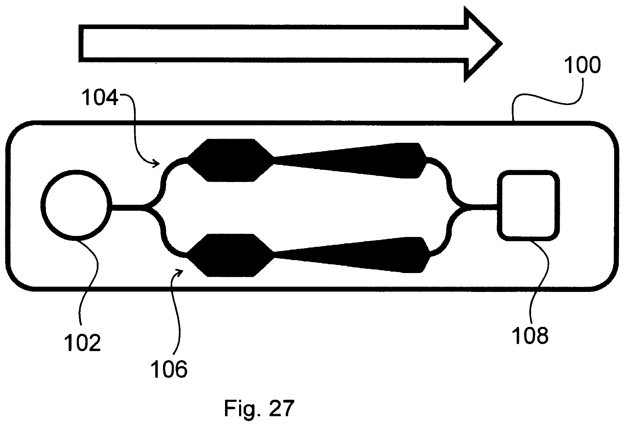

By way of example, Fig. 27 shows a sample distributing part that can be applied in the capillary channel system according to the invention. This sample distributing structural member is also called a sample gate. The sample distributing part (sample gate) may be, for example, a chamber or a container or a suitably formed part of the surface, e.g. a surface indentation. The shape of the sample gate must ensure the uniform distribution of the liquid into the channels and also the simultaneous introduction of the sample into the channels.

When the capillary channel or capillary channel system according to the invention is applied in a method according to the invention, the sample is preferably blood, the colour of which in front of an appropriate background enables easy visual tracking. Preferably, by stopping the liquid flow at an appropriate time, constant, unchanged and measurable liquid endpoints are formed within the reader section.

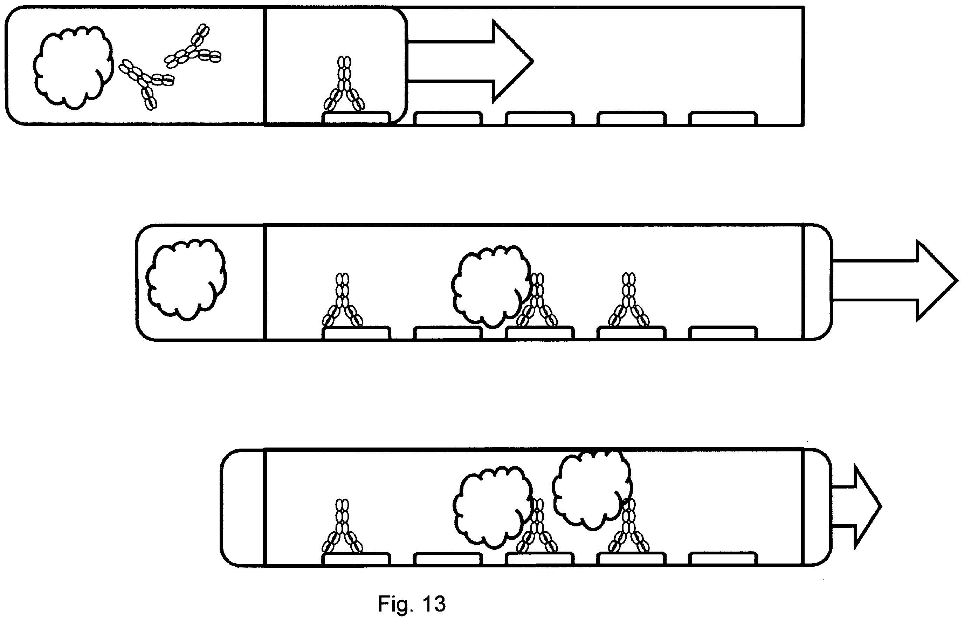

The phenomenon occurring in the trap is illustrated schematically in Fig. 13, in the case when an antigen-containing capillary channel of the capillary channel system according to the invention is used in a method according to the invention. In the trap section, (1) the antibodies bind from the flowing sample to the antigen applied (bound) to the inner surface of the capillary channel or to the outer surface of the constricting element (optionally to ground layer), (2) and then the cells adhere to the antibodies and to the generated immune complexes, respectively, after the binding of an appropriate quantity and quality of antibody; (3) cell adhesion slows down the rate of the liquid flow.

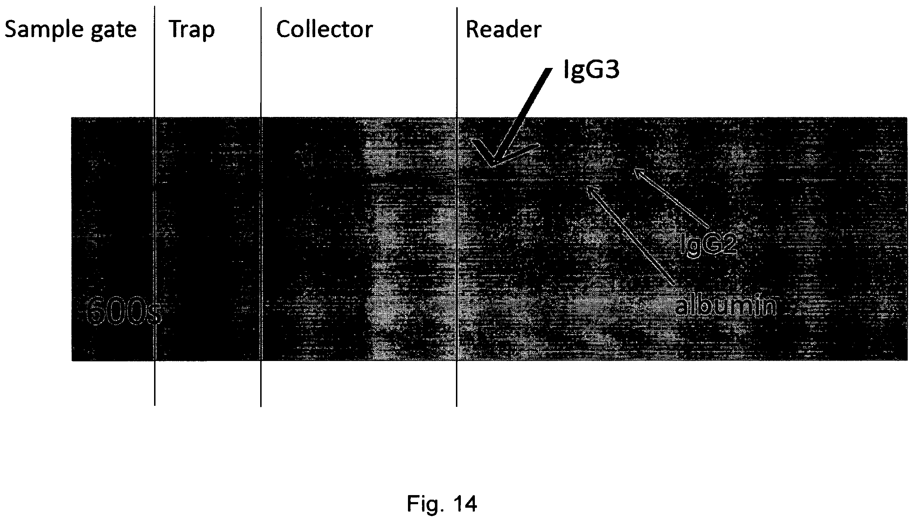

Fig. 14 shows the slowed down liquid flow: three capillaries are used, wherein the trap of each capillary comprises a different material (albumin, IgG2 or IgG3), and the blood flowing in the capillary channels is tracked by naked eye. After 10 minutes, the liquid has covered a shorter distance in the case of IgG3 ensuring cell adhesion.

Capillary channel systems can be formed also by using rules, materials, methods and apparatuses known in the science of microfluidics. Routes characterised by widths and/or depths varying in the micrometer range can be formed in solid materials, for example in silicon panels or glass sheets, by various micromachining procedures, for example by means of laser or DRIE (deep-reactive ion etching) technology. The material (substrate) of the microfluidic device may have one or more components. The capillary channel system may be formed e.g. by coating a glass surface with antigens and reference (control) materials, and then coating the glass surface by a polymer comprising pre-formed channels, preferably a silicone elastomer (PDMS), thereby making routes. A chamber communicating with the channel system formed in the polymer layer serves as a sample gate. Similarly, when glass and elastomer are used to form a capillary channel system, constricting elements with a coating, preferably an antigen coating, may be fitted therein, hence creating a trap. A microfluidic device adapted to implement the methods according to the invention may also be made by using plastics (polymers).

Preferably, in the case of an RDT device, the sample gate, as a result of its configuration and volume, is suitable for receiving blood drawn from the fingertip in the form of a blood drop, and accordingly for receiving a sample of 1 to 100 microliter, preferably a sample of 2 to 50 microliter volume.

When the capillary channel system according to the invention is used for implementing a method according to the invention, the sample gate takes up a sufficient volume of the sample to be tested so that the sample flowing into the channels during the measurement is replenished on an ongoing basis until the moment of reading. Accordingly, the sample gate is suitable at least for receiving a sample volume corresponding to the combined volume of traps plus collectors plus readers. In a preferred embodiment, the measuring channels and the associated negative and positive control channels have a common, or at least interconnected sample gate, because the measurement is based on comparing the flow of the sample in the various channels.

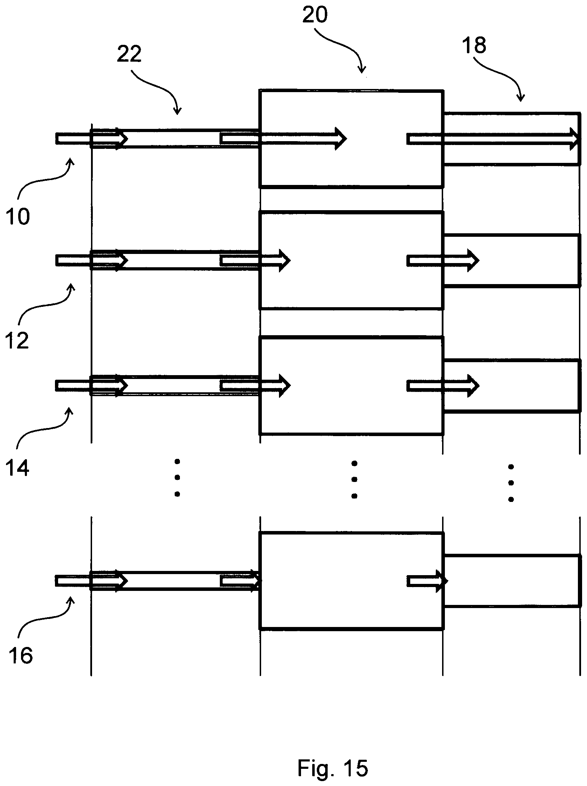

Fig. 15 is a schematic illustration of a capillary channel system according to the invention, in other words a capillary channel array (i.e. an array of capillary channels), which comprises a negative control channel 10, a positive control capillary channel 16, and antigen-containing measuring channels 12, 14. The so created capillary channel array (capillary array) is a multi-feature antigen array and it is adapted for implementing the methods according to the invention. For the measurement, an array comprising one or more antigen-containing capillary channels (e.g. the measuring channels 12, 14) and one or more control channels (e.g. in the form of capillary tubes; the negative control capillary channel 10 and the positive control capillary channel 16) is used, and the capillary channel comprising the negative control ('negative control channel') allows free flow in the trap section, thereby enabling the fastest liquid flow - identical with or faster than the liquid flow in the measuring channel. The capillary channel comprising the positive control ('positive control channel') provides very efficient cell adhesion, thereby allowing a slow liquid flow - identical with or slower than the liquid flow in the measuring channel. The measuring channel is a capillary channel, which comprises the relevant antigen, i.e. the one at which the analysis is aimed; the capillary channel array may comprise one or more measuring channels. In the measuring channel, preferably such an antigen is applied as an antigen, which is able to adhere specifically to such an antibody which is specific to a certain condition or disorder. Preferably the analysis is aimed at detecting antibodies of biomarker value, i.e. diagnostic antibodies, the presence of which characterises the immunological status (infection, protection) or pathogenic condition (autoimmunity, allergy, tumour). The liquid flow, e.g. the flow rate is determined by the interactions of the antigens, the antibodies of the test sample and the cells of the test sample. The flow rate relative to the negative and positive controls, and observable or measurable in the reader section shows the serologic reactivity of the sample tested, in relation to the antigen present in the measuring channel. Fig. 15 shows a trap section 22, a collector section 20 and a reader section 18 of the identical channels 10, 12, 14, 16.

In the measuring channel, connective tissue proteins or bacterial superantigens are not used as antigens. At the same time, these antigens can be used in the trap of the positive control channel. In certain embodiments, the control material applied in the coating of the trap of the positive control channel is selected from the group consisting of superantigens, connective tissue proteins and materials other than antigens. In the negative control channel, the control material used as a coating of the trap is a material which does not function as an antigen, and it is typically an albumin, preferably BSA (bovine serum albumin). In certain embodiments, the control material applied in the coating of the trap of the negative control channel is selected from the group consisting of albumins and materials other than antigens. The configuration of the positive and negative control channels, e.g. the geometry (constrictions and enlargings, etc.) is identical with those of the associated measuring channels.

In case there is no coating in the trap of the negative control channel, the reader of the negative control channel is defined in a way that the reader is positioned in the negative control channel in the same place within the channel as the reader in the measuring channel.

In some embodiments, the flows occurring in several capillary channels (positive control, negative control, measuring channels) can be tracked in the reader sections simultaneously. Optionally, the various measuring capillary channels may differ in the configuration of their traps and collectors in order to ensure that a valuable liquid-flow pattern is established in the reader sections, from the aspect of the measurement. In this embodiment, an individual control channel or individual channels is/are associated with each differently formed measuring channel.

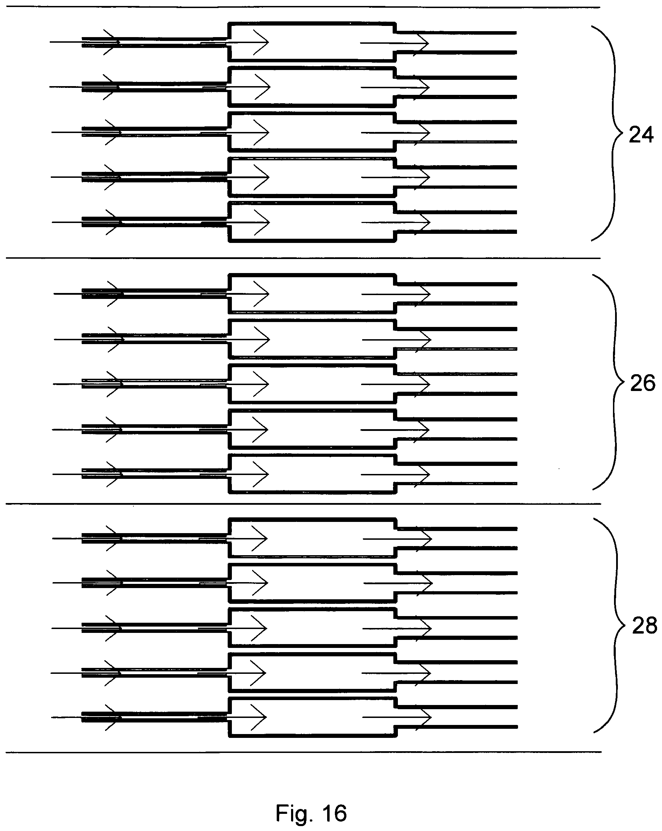

Fig. 16 is a schematic illustration of a capillary channel system (capillary array) according to a further embodiment of the invention. When such a capillary array is applied, simultaneous measurements are carried out in several identical channels, and, therefore, semi-quantitative results that can be characterised also statistically are obtained from the measurement. In this embodiment of the invention, e.g. capillary channels 24 represent a negative control, capillary channels 26 represent the measuring channels, and capillary channels 28 represent the positive control. In the present embodiment, each group (group of negative controls, group of measuring channels, group of positive controls) comprises five identical capillary channels; the results of five channels in each category can be already subjected to a statistical test.

In an embodiment of the capillary channel system according to the invention, several measuring channels of an identical structural configuration may be applied, with their antigen coatings being different from each other. In this case, a common negative control channel and/or positive control channel may be associated with the several measuring channels. Accordingly, in this embodiment, the number of negative control channels and/or positive control channels is lower than the number of measuring channels. In other embodiments, however, the numbers of measuring channels, positive control channels and negative control channels are identical.

Preferably the whole structure, i.e. the device comprising the capillary channel system is of a few centimeters lateral size, but the length of the capillary channels may be longer than the length of the whole structure in an appropriately formed microfluidic system. The invention extends to all capillary channels and capillary channel systems, falling within the scope of the claims, which are made with a material, shape and structure that ensure as a result of the processes described above that the antibodies of given specificities of a biological sample can be measured by means of changes in the liquid flow resulting from the interaction between the antibodies bound to the antigen and cells present in the sample.

When the capillary channel or channel system according to the invention is applied for implementing the method according to the invention, in an embodiment the reading of the result of the measurement, i.e. the determination of the flow characteristic, comprises reading the distance covered by the sample, preferably blood, over a certain period of time, and preferably comprises reading the distance covered by the sample, preferably blood, over a certain period of time in the negative control channel and in the one or more measuring channels and the comparison of these readings. In an embodiment, it is ensured that the measurement is started simultaneously in the negative control channel and in the one or more measuring channels, i.e. that the sample enters the channels simultaneously. This can be achieved, for example, by using a capillary channel system according to the invention, which has a sample distributing part. In this embodiment, the determination of the flow characteristic is carried out for example by observing/reading in the reader section where the liquid front in the measuring channel is currently located as compared to the liquid front (the very first part of the blood flow) in the negative control capillary channel. Alternatively, the determination of the flow rate comprises measuring the distances covered by the liquid front of the sample in the various channels per unit time. Optionally, the obtained data are compared.

If the measurement is not started simultaneously in the various channels, then the determination of the flow characteristic typically comprises measuring the distance covered in one unit of time by the liquid front of the sample in each channel.

From the distance covered by the liquid front of the sample in the measuring channel (optionally per unit time), a conclusion is drawn about the reactivity of the sample to the antigen located in the measuring channel. If the liquid front of the sample covers a shorter distance in the measuring channel (optionally per unit time) than in the negative control channel, the sample exhibits reactivity to the analysed antigen present in the measuring channel. The comparison may be made e.g. at a time falling into a time period starting from the moment when the sample both in the negative control channel and in the measuring capillary channel has already reached the reader section, and ending with the moment when the liquid front of neither sample in these channels has left this section yet.

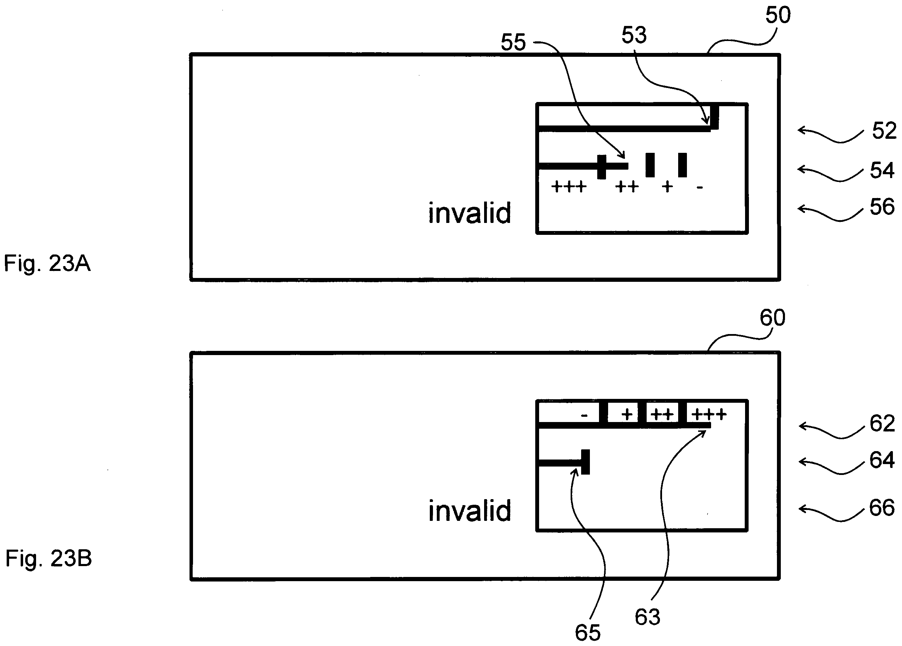

Figs. 23A and 23B illustrate two preferred reading methods of the result. In Fig. 23A, in the capillary channel system according to an embodiment of the invention and arranged on a slide 50 (object-plate), a liquid front 53 of the sample proceeds further in a negative control channel 52 than a liquid front 55 of the sample in a measuring channel 54. In the example of Fig. 23A, the liquid front 53 of the sample flowing in the negative control channel 52 reaches a predetermined (fixed) point in the reader section. This fixed point is marked with a vertical line in the figure; the measurement ends when the liquid front 53 of the sample reaches this point. This means that the capillary channel system is calibrated in a way that the flow of the sample is slowed down in the measuring channel 54 to such an extent that when reaching the fixed point in the negative control channel 52, the capillary channel system shows the appropriate result. Fig. 23A depicts a relatively strong (++) positive result.

Another reading method is illustrated in Fig. 23B. In the case shown in the figure, a liquid front 65 of the sample flowing in a measuring channel 64 of the capillary channel system according to a further embodiment and arranged on a slide 60 reaches a predetermined (fixed) point of the reader section; the measurement ends when the liquid front 65 reaches this point. In this case, contrary to the example in Fig. 23A, the present embodiment of the capillary channel system is calibrated in a way that the flow of the sample slows down exactly to such an extent in the measuring channel 64 that when reaching the fixed point, exactly the appropriate result appears in a negative control channel 62, i.e. a liquid front 63 reaches exactly the appropriate range. Then, in the negative control channel 62, the measurement falls into the section marked by the markings +++, i.e. the result of the measurement shown in Fig. 23B is very strongly positive.

In Figs. 23A and 23B, liquid front of the sample fails to appear either in a reader of a positive control channel 56 or in a reader of a positive control channel 66, which means in the present embodiment that the measurement is valid (if the sample appeared in the positive control channel, the measurement would be invalid, as shown by the 'invalid' caption in Figs. 23A and 23B).

By identifying the liquid front of the flowing sample in relation to the preestablished markings of the reader section (i.e. taking a reading of the position of the liquid front of the flowing sample) it can be determined whether the flow rate significantly differs in the measuring channel from the flow rate observable in the negative control channel. In the reader sections, the signals are typically positioned depending on the type of antigen(s) in the measuring channel.

Even more preferably, the capillary channel system is formed in a way that the flow stops when the blood flowing in the negative control channel or in the measuring channel reaches the end of the reader, and thereby the results are registered in the readers, with the option of taking the reading later on.

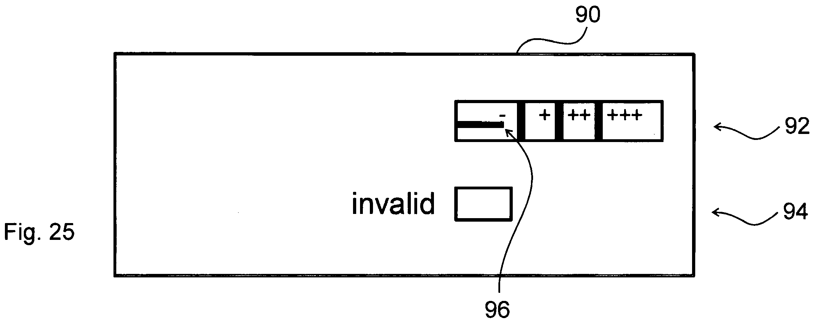

A capillary channel system according to such an embodiment is shown in Fig. 25, where on a slide 90 there are two windows showing a negative control channel 92 and a positive control channel 94. In this case the capillary channel system provides a result, if the sample reaches the fixed point of the measuring channel - which is concealed (has no window) in the present embodiment - and thereby triggers automatic stopping of the flow in the negative control channel 92, i.e. the present embodiment is similar to the arrangement shown in Fig. 23B. The measuring result shown in Fig. 25 is negative, because a liquid front 96 of the sample only reaches the (-) zone associated with a negative result. The measurement is valid, because the sample does not appear in the reader of the positive control channel 94.

The result of the analysis can be considered to be positive, i.e. immune reactivity can be ascertained (found) regarding the tested antigen, if
a) there is a statistically significant difference between the flow rates measured/observed in the measuring channel and in the negative control channel, and
b) positive control, if applied, works appropriately (i.e. in certain embodiments the sample does not appear in the field tagged 'invalid').

The appropriate working of the positive control means that an area marked in the reader (the so-called validation area which is tagged 'invalid' in Figs. 23A-25) is not reached by the sample in the positive control channel during the test. If the sample in the positive control channel reaches the validity area, the measurement is invalid and can not be evaluated. Such a case is shown in Fig. 24B. Fig. 24B depicts a measurement performed on a slide 80. The measurement shown in Fig. 24B comes to an end when reaching a fixed point located in a measuring channel 84. Fig. 24B shows the status similarly to Figs. 23A-25 when the measurement is already completed. It can be seen that in a negative control channel 82, a liquid front 83 of the sample, in the measuring channel 84 a liquid front 85 of the sample, and in a positive control channel 86 a liquid front 87 of the sample practically cover distances identical with each other and this indicates that the antigens in the measuring channel do not influence the velocity of the sample. The sample also appears in the reader of the positive control channel 86 which indicates the invalidity of the measurement. This could be caused by an inappropriate introduction of the sample, an error of the device or a pathologically low number or pathologically inferior adhesion ability of the tested individual's cells. The validity area marked in the reader is formed and determined by measuring a large number of samples.

Fig. 24A shows a valid measurement. In this measurement, a liquid front 73 of the sample in a negative control channel 72 and a liquid front 75 of the sample in a measuring channel 74 practically cover the same distances, i.e. the antigens in the measuring channel do not influence in this example the flow of the sample to a measurable extent, i.e. the result of the measurement is negative. In the present case, the sample does not appear in a positive control channel 76, and, therefore, the measurement is valid.

When flow can be considered as being significantly slower than the one for negative control is determined by measuring a large number of samples. By measuring samples determined by other methods to show a negative (i.e. no) or positive reaction to the antigen tested, the shortest distance is marked in a given capillary array between the liquid front of the negative control channel and that of the measuring channel, which represents a positive reaction with a given reliability (for example, 95%). This difference may be marked in the area of the reader. The larger the distance between the liquid front of the negative control channel and that of the measuring channel, the more positive the reaction can be considered to be. To characterise this, optionally further areas are marked in the reader section (for example, +, ++, +++).

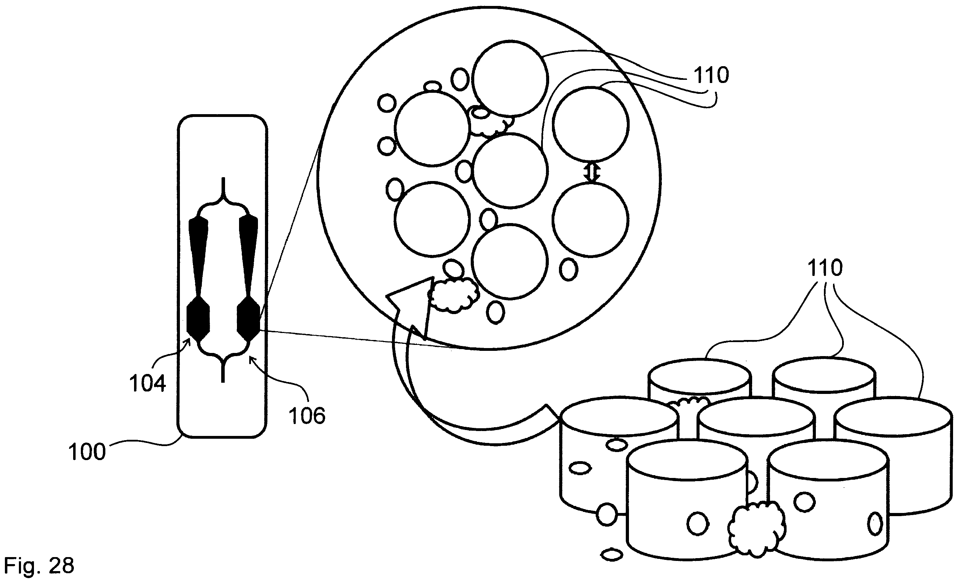

A further embodiment of the capillary channel system according to the invention is shown in Figs. 27 and 28. In the present embodiment, the capillary channel system is arranged on a slide 100, and it comprises a negative control channel 104 and a measuring channel 106 (a capillary channel as defined in claim 1). The negative control channel 104 and the measuring channel 106 are formed as so-called bifurcating channels. This means that the two channels have a common sample distributing part 102, which then branches off into two branches (the negative control channel 104 and the measuring channel 106). The two branches are reunited at the end of the negative control channel 104 and the measuring channel 106, where a container 108 is arranged.

It is a great advantage of the present embodiment that it automatically ensures the stopping of the sample in one of the channels. This is because in case the liquid front of the sample reaches in the negative control channel 104 or in the measuring channel 106 the meeting point of the two channels, the capillary forces are no longer able to overcome the pressure of the air column remaining in the tube (a closed air column is formed) and the liquid front stops in the other channel where the liquid front is further back. Accordingly, the meeting point of the two channels will be the fixed point, and along the other channel the markings (-, +, ++, +++) necessary for the evaluation of the measurement may be arranged with an appropriate calibration.

Thus, in the present embodiment, the capillary channel system comprises a single measuring channel and a single negative control channel, and the measuring channel and negative control channel are connected with their end points to a common draining capillary channel.

In Fig. 27, the arrow shown along the slide 100 indicates the flow direction of the sample.

Fig. 28 shows a magnified view of the trap section of the embodiment of Fig. 27. It is depicted by the magnified part of Fig. 28 that the trap section is formed by arranging inner constricting elements 110 in the inner space of the appropriate section of the capillary, and said constricting elements ensure that the trap section only has traversable cross-sectional areas with appropriately narrow characteristic size, preferably of less than 20 micrometers and even more preferably of less than 10 micrometer. It is also shown by the magnified parts of the figure that the inner constricting elements 110 are of a cylindrical shape.

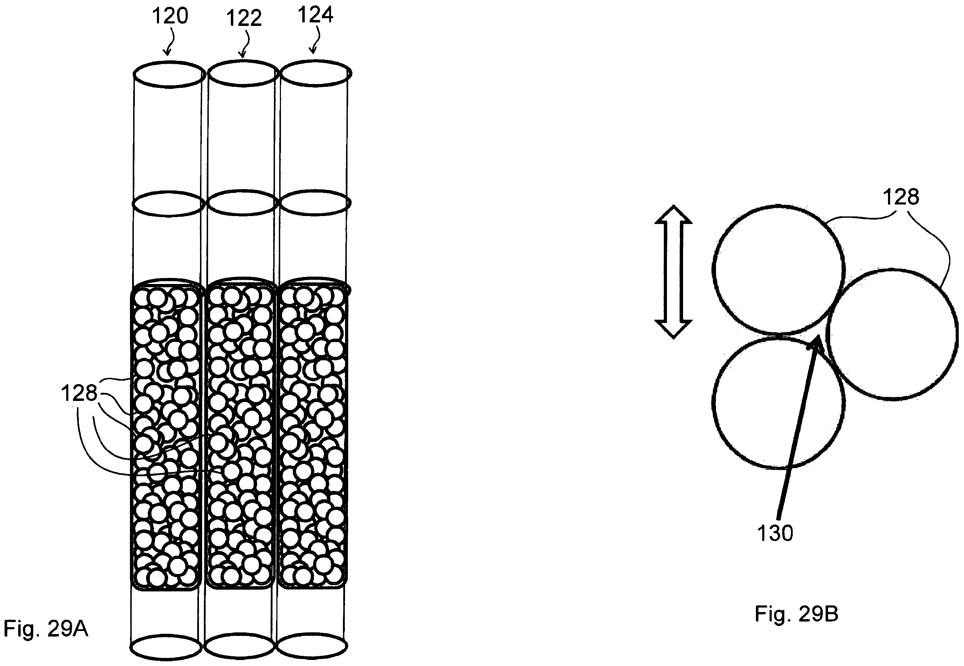

Fig. 29A shows a capillary channel system comprising three capillaries 120, 122, 124, in the capillaries spheres 128 are arranged as constricting elements in the trap section as shown in Fig. 29A. Fig. 29B illustrates that by this arrangement of the spheres 128 it can be ensured that the traversable cross-sectional areas are of a limited size, i.e. their characteristic dimensions e.g. should be preferably between 3 and 30 micrometers (by the appropriate selection of the size of the spheres 128, the desired characteristic size matching to the given test may be adjusted). The diameter of the spheres 128 is typically between 20 and 200 micrometer.

In a further embodiment, a reader is used which comprises physical and/or chemical sensor(s) adapted for the quantitative measurement of a characteristic of a liquid flow, for example the flow rate. In this case, the rate of the liquid flow is directly measured in the individual channels (in the measuring channels and the control channel(s)), and the results obtained are compared on the basis of the discussion above, to draw a conclusion about the reactivity of the sample to the antigen present in the measuring channel.

For implementing the methods of the invention, e.g. a diagnostic method according to the invention, by using capillary channels or capillary channel systems according to the invention, such as for example capillary tubes, preferably one drop of sample, preferably blood is used. Taking into consideration that for example a capillary tube typically takes up a quantity of around 1 microliter and that the volume of a blood drop is approximately 50 microliter, from one blood drop 10-plex measurements may also be carried out (i.e. a method which covers 10-plex measurements), i.e. with three parallels, 10 types of measuring channels, and one positive and one negative control.

Typically, the reading of the result is performed or optionally the flow rate is measured 1 to 30 minutes after starting the running of the sample.

In capillaries the force which moves the liquid is determined by the angle enclosed by the liquid and the capillary surface, as well as the curve of the meniscus of the liquid front (contact of liquid-solid capillary wall and gas phases). The liquid wetting the capillary moves forward as a result of this capillary pressure, as long as this force exists. Practically, in a capillary system, the liquid moves as long as it is replenished, and the force acts in a member of the system, filling said system up. Those microfluidic systems in which the liquid(s) move(s) merely as a result of the capillary forces are called capillary autonomous systems (CAS) [Autonomous microfluidic capillary system. Juncker, D. et al. Anal. Chem. 2002, 74, 6139-6144.]

In autonomous capillary systems, valves [Valves for autonomous capillary systems. Zimmermann M et al. Microfluid Nanofluid 2008. 5:395], pumps [Zimmermann, M. et al. Capillary pumps for autonomous capillary systems. Lab Chip 2007. 7:119], chambers [Clime, L. et al. Self-priming of liquids in capillary autonomous microfluidic systems. Microfluid Nanofluid 2012. 12:371] for regulating the movement, path and interaction of the liquid may be fitted implementing the regulation of complex processes.

In a preferred embodiment, the capillary channels form an autonomous capillary system and valves fitted in the reader section ensure that the flowing of the sample stops when the reading of the distance covered in the negative control channel and that in the measuring channel can be performed and the result can be evaluated.

### Applications

Characterisation of immune responses against prion, viral, bacterial, fungal or parasitic infections following a vaccination is of crucial importance in the clinical practice. Antigen microarrays have been successfully used for serodiagnosis of infectious diseases (Mezzasoma et al., Clin. Chem. 2002, 48:121-130). In another embodiment, the methods of the present invention are useful for evaluating the nature of the immune response with respect to the cell activating properties of the induced antibodies.

Obtaining biologically relevant information in antigen array experiments is important in a number of applications from screening autoimmune sera to assessing vaccination efficacy; therefore, the method has widespread potential uses.

An instance of the method of the disclosure is a method for the detection of components (e.g. antibodies) in a sample, e.g. body fluid or solution, on an antigen array formed of capillary channels, preferably on an antigen microarray, which components have specific reactivity to antigens, the method comprises immobilizing the antigens on a support, thereby forming capillary channels; contacting the sample (e.g. body fluid or solution) comprising said components with the antigen array, preferably by passing the sample through the antigen array; optionally adding cells thereto, and revealing and/or measuring the bound (adhered) cells or cellular responses of the cells; wherein the cells are optionally labelled.

If the methods of the disclosure, e.g. the method according to the above described preferred instance, is targeted for the analysis of antibodies present in the sample, then antibody tests are performed.

Antibody tests are methods for the measurement of antibodies having a particular recognition ability (specificity). The detection of the presence and amount of antibodies of a particular specificity in the circulation is of diagnostic value in a number of pathological conditions.

The detection of antibodies recognising pathogens may serve for recognising an infection, while the qualitative determination of said antibodies reflects immunity following vaccination. While in the early period of the infection the detection of the antigens of the pathogen is diagnostic, after the development of the immunity the appearing pathogen-specific antibodies indicate efficiently the ongoing or earlier infection. Vaccinations must induce the production of antibodies against the respective pathogen, however, as vaccination does not work with an efficacy of 100%, testing the appearence (presence) of antibodies is of importance.

Detection of antibodies forming against the self components of the body is the basis of diagnosing various autoimmune diseases. These autoantibodies may impair the organism, or may have indicative values, only, but the serological tests are necessary elements of diagnostics by all means. Autoantibodies may appear also in tumour diseases and they also may facilitate diagnosis and/or classification.

Allergy is excessive immunoreactivity against environmental antigens; the detection of antibodies which recognise allergens assists in the precise characterization of the disease. The appearance of IgG antibodies recognising certain components of the food may lie behind food allergies.

Table 1 below gives examples for antibody-tests, which, however, the present application is not limited to the tests shown. Thus, in the Table below only some examples are provided as to which antigen or antigens can be used in the methods according to the invention, e.g. to diagnose certain diseases or conditions. The skilled person will readily appreciate that a large number of diseases exist for which the detection of antibodies is of diagnostic value. The antigens listed in Table 1 can be also preferably used in the measuring channels of the capillary channel systems according to the invention.

**Table 1**

| **Immunity against a pathogen due to infection or vaccination** | **pathogen** | **examples of antigens used according to the invention** |
|---|---|---|
| | Hepatitis B virus | HBsAg, HBcAg |
| | Clostridium tetani | tetanus toxin |
| | Plasmodium falciparum | CSP |
| | mumps | infected cell-lysate |
| | poliovirus | viral protein peptides |
| | influenza virus | HA, NA |
| | HIV | gp120, gp41, p24 |
| | Mycobacterium tuberculosis | Ag85 |
| | Borrelia burgdorferi | Flagellar antigen |
| | Streptococcus pyogenes | Streptolizin, DNase B, hyaluronidase |
| | Epstein-Barr virus | EBNA |
| | CMV | CMV early and late antigens |
| | Dengue virus | DV envelope proteins |
| | Salmonella typhi | Whole bacterium |
| | Rotavirus | Whole virus |
| | HSV1 | Envelope glycoprotein G |
| | Bacillus anthracis | Protective antigen |

| | **disease** | **source of antigen** |
|---|---|---|
| | joint infection | N. gonorrheae, S.aureus, B.burgdorferi, Brucella species, P. aeruginosa, P. multocida, E.corrodens, S. moniliformis, M.hominis, U.urealyticum, Mycobacterum species; HBV, RV, MV, LCV, HIV; Sporothrix -, Candida, Coccidioides species |
| | Chronic meningitis | Brucella species, B. burgdorferi, T. pallidum, Mycobacterium species, Nocardia species, Actinomyces species; Candida species, Coccidioides immitis, Histoplasma capsulatum, Cryptococcus neoformans, Sporothrix schenckii |
| | Genital infection | Neisseria gonorrhoeae, Neisseria meningitides, Chlamydia species, Actinomyces species, Treponema pallidum, Haemophylus ducreyi, Klebsiella granulomatis, Mycobacterium species, Gardnerella vaginalis, Mycoplasma hominis; HSV, CMV, Adenovirus, papillomavirus; Histoplasma capsulatum |
| | prostatitis | E.coli, Klebsiella species, Proteus mirabilis, Enterobacter species, Enterococcus species, Mycobacterium species, Neisseria gonorrhoeae, Cryptococus neoformans, Candida |
| | bronchitis | Mycoplasma pneumonia, Chlamidophyla pneumonia, Bordetella pertussis, Moraxella ctarrhalsi, Haemophilus influenzae; rhinoviruses, coronavirus, parainfluenzavirus, influenza virus, RSV, adenovirus |
| | sinusitis | S. pneumonia, H.influenze, M. catarrhalis, S.viridans, S.aureus, S.pyogenes, C.pneumoniae, P.aeruginosa, rhinovirusis, influenzavirus, parainfluenzavirus, adenovirus, Aspergillus species, Hyphomycetes, Zygomycetes |

| **development of autoantibodies** | **disease** | **antigen (autoantigen)** |
|---|---|---|
| | systemic lupus | dsDNA, SSA, CL, histon |
| | rheumatoid arthritis | citrullinated vimentin, -collagen, -fibrin |
| | autoimmune diabetes | ICA, insulin, GAD |
| | tumour | tumour antigens |
| | scleroderma | Topoisomerase, CENP-B |
| | Wegener's granulomatosis | ANCA |
| | dermatomyositis | Jo-1 |
| | Sjögren's syndrome | SSA, SSB |
| | Autoimmune hepatitis | LC-1, F-actin |
| | myasthenia | AChR |
| | cardiomyopathy | Beta-1 adrenergic receptor |
| | pemphigus | desmoglein |
| | Anaemia perniciosa | PCA |
| | APS | phospholipids |
| | coeliakia | endomisium |
| | Sclerosis multiplex | MBP, MOG |
| | NMO | Aquaporin |
| | Primary biliary cirrhosis | AMA M2 |
| | Wegener-granulomatosis, CS-syndrome, polyarteritis | ANCA |
| | Neuropathy, Guillain-Barré syndrome | myelin |
| | atherosclerosis | Oxidized LDL |
| | thyroiditis | thyroglobulin |
| | Based ow-G raves disease | TSH |
| | infertility | sperm |
| | ITP | platelet surface antigens |
| | Hemolytic anemia | RBC antigens |

| **excessive immunoreactivity** | **disease** | **antigen (allergen)** |
|---|---|---|
| | celiac disease | gluten |
| | sensitivity to egg | albumen |
| | food dye allergy | dyes |

Often the detection of a single sort of antibody is not sufficient for the diagnosis or the characterisation of the immune status. Multiplex antibody detection/tests, also called immune profiling can provide a more complete or more precise characterisation of a condition.

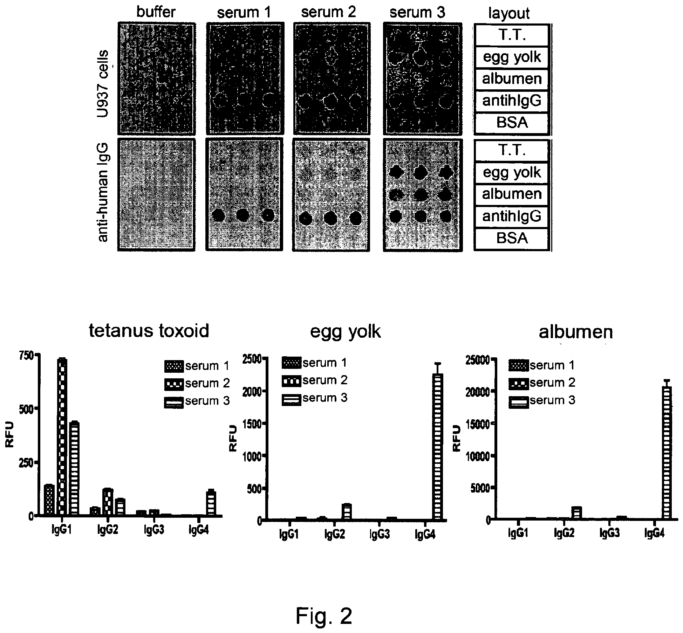

With the methods of the present invention (capillary based microarray technique), reactions with more than one antigens can be simultaneously tested (analysed) and this is advantageous in the testing for diseases. The above-described experimental conditions represent a good approximation to natural conditions. During the treatment of the antigens with the sample, e.g. serum, antibodies with different isotypes but identical specificity, and other soluble molecules recognising the antigen in question will compete for the antigen, and a highly complex immune complex may be formed, and this method is useful for the analysis of the combined effects (cell adhesion, activation) of these molecules. Figure 2 demonstrates that multiple antibody-subclasses may bind to a single antigen.
In the case of pathogens the aim of multiplex tests may be e.g. differential diagnostics. For example, in the case of an infection of the upper respiratory tract, immune responses indicating the presence of various potential pathogens (e.g. influenza virus, coronavirus, Haemophilus influenzae) can be tested simultaneously, thereby confirming some pathogens and excluding others in the diagnosis. Within a given bacterial species serotypes can be differentiated, and, in the case of viruses, strains, variants; by their simultaneous testing more precise diagnosis can be established. A further option is to test the components of a given pathogen separately (several different proteins), and the analysis of several epitopes simultaneously, respectively, by which the nature and efficiency of the immune response, and its accidental adverse effects can be assessed.

In the case of systemic autoimmune diseases autoantibodies of various compositions may appear, and, therefore, by testing simultaneously several antigens differential diagnostic data may be obtained.

In allergy tests, the combined testing of several candidate allergens may facilitate the characterisation of the disease, e.g. to assess the spreading of allergenic epitopes.

It follows from the above that the use of the devices according to the invention and/or of the methods according to the invention has a number of advantages over that of the known technical solutions, as the present one provides information about the effects of the antigens exerted on cells in the individual examined (i.e. the donor of the sample); it provides semiquantitative results reflecting also the potency of the reaction; it is suitable for testing (analysing) reactivity to multiple antigens simultaneously; there is no need for detection antibodies produced in animals, and it can be readily integrated for instrumental measurements.

The invention can be advantageously used in agriculture, e.g. in animal farming, veterinary applications, as well.

It is to be appreciated that many conventional steps and procedures, including e.g. blocking and washing steps, occurring during the implementation of the methods of the present invention, are not described herein. These steps and procedures are well known to those in the art.

The following examples are given as further illustrations of the present invention and should not be interpreted as limitations of the scope of the invention.

### EXAMPLES

### Methods

### Cell lines, and loading cells with fluorescent dyes

U937 (ATCC Number: CRL-1593.2) (38) monocyte cell lines were maintained in an incubator at 37°C and 5% CO₂, in polystyrene cell culture flasks containing an RPMI-I medium supplemented with 10% FCS.

The desired quantity of cells was centrifuged at 1200 rpm for 7 minutes, the supernatants were discarded, and after loosening the pellet, the cells were resuspended at a concentration of 10⁶ cells/mL in empty RPMI-I containing 4 µM Celltracker Green (CMFDA, Invitrogen), and then incubated for 30 minutes with shaking at 90 rpm and under protection from light exposure. Next, cells were centrifuged again, and were incubated in RPMI-I containing 10% FCS and 10⁷ cells/mL for 30 minutes under shaking at 90 rpm and under protection from light exposure. Cells were passed through a cell filter, then centrifuged and adjusted to the desired cell count using RPMI-I containing 10% FCS.

### Preparation of antigen microarray

Small volumes of the antigens, proteins selected for the test (analysis) were printed in triplicate or sextuplicate spots per subarray to nitrocellulose membranes attached to slides or to FAST Slides (Whatman) coated with 16- or 2-subarray nitrocellulose purchased from the manufacturer, using a BioOdyssey Calligrapher Miniarreyer (Bio-Rad) chip printer. Until use, the protein chips thus prepared were stored in bags ensuring protection from light and moisture, at 4°C.

### Measuring and evaluating the antigen microarray

Treated chips were read using an Axon GenePix 4300A (MDS Analytical Technologies) scanner with an optical filter and wavelength suitable for the dye measured, and with amplification. Evaluation was performed using the GenePix 7 pro (Axon) software program. The raw data thus generated were processed in MS Excel.

### Example 1

### Detection of different antibody subclasses via detection antibodies to human IgG, complement C3 fixing ability and adhesion of U937 monocytoid cells

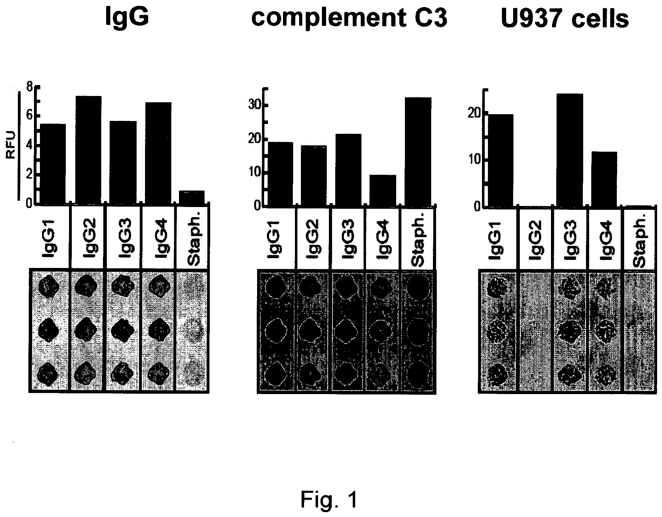

Human immunoglobulins belonging to four different subclasses (IgG1, IgG2, IgG3, IgG4) were printed onto nitrocellulose arrays in a concentration of 0.5 mg/mL, using solid pins with a diameter of 300 µm. These IgG subclasses are known to possess diverse IgG FcR-binding and complement activating abilities. To test the ability of monocytes to bind to antigens independently of antibodies, *Staphylococcus* bacteria were also printed. Slides were developed by three different methods (Figure 1). First, FITC-labelled monoclonal murine antibody against human IgG (Invitrogen, CA) was diluted to 500-fold in array buffer (5% BSA, 0.05% Tween 20 in PBS), and applied to the array for 60 minutes at 37°C for IgG detection. Second, fresh serum was applied as a complement source for 1 hour at 37°C, then bound complement C3 fragments were detected by Alexa-647-labelled polyclonal goat anti-human C3 antibodies (MP Biomedicals, OH) in a dilution of 1:5,000 in array buffer. Third, U937 monocytoid cells were loaded with CellTracker Green (4 µM dye in RPMI 1640 medium, 10⁶ cell/mL for 30 minutes at 37°C) and applied to microarrays at a concentration of 1 million cells per chamber in 100 microliters of an RPMI 1640 medium containing 10% FCS. Slides were kept in a humidified atmosphere in an incubator at 37°C, with continuous slow shaking. All three slides were finally washed 3 times in RPMI 1640 and PBS containing 0.05% Tween 20 in case of cells and detection antibodies, respectively. Chips were then scanned with Axon Genepix 4300A at a resolution of 5 µm. Columns stand for means of triplicates from one out of three representative experiments. These detection methods gave strikingly different results. The detection antibody against human IgG detected all four subclasses with comparable sensitivity. The detection of complement C3 fragments revealed the different complement activating potential of the different subclasses of IgG, IgG4 being the most inert in this respect. With the resolution used, individual cells could be detected on the array; approximately 2,000 cells covered the whole antigen spot. The fluorescence intensities of the adhered cells reflected the known potential of IgG1 and IgG3 to bind to Fc receptors. Importantly, the detecting reagent against human IgG did not indicate these differences in biological activity. Very strong complement activation but practically no cell binding above the background was observed on printed *Staphylococcus* bacteria, implying that not all receptors on the cells are capable of inducing cell adhesion.
Staph = Staphylococcus suspension

### Example 2

### Detection of serum antibody binding patterns

In order to confirm that not only printed antibodies but also antibodies bound to printed antigens can be efficiently detected by monocytes, common antigens such as tetanus toxoid (T.T.), albumen and yolk, as well as serum samples with known reactivity against these antigens were selected. These antigens were printed on nitrocellulose along with IgG capture antibodies as positive controls and bovine serum albumin (BSA) as negative control. Microarray slides were incubated either with buffer only or with one of three serum samples with different reactivities (1:5 dilution in veronal buffer containing 20 mM EDTA and 5% BSA). Two parallel reactions were set up for each condition and one was probed with anti-human IgG, and the other with U937 cells loaded with CytoTracker. Images were generated as in Example 1 (Figure 1). As the upper part of Figure 2 shows, detection by cells was comparable or even more sensitive than detection by secondary reagents. The tetanus toxoid reactivity of the serum samples was detected by cells but not by antibodies. Interestingly, detection antibodies could reliably detect albumen-specific IgG in Sample 3 even though it was comparable to tetanus toxoid reactivity. This finding highlights the differences between the two detection systems, which are attributable to the broader recognition ability and yet selective nature of cellular detection.

The lower diagrams of Figure 2 show the IgG subclass distribution for the three antigens. The antigen arrays were detected with subclass-specific murine monoclonal antibodies, and relative fluorescence intensity (RFU) is represented on the axis. It is visible that in individual sera, IgGs of more than one subclasses react with a given antigen. In addition, it can be observed that anti-tetanus toxoid antibodies are primarily of the IgG1 types and anti-egg yolk and anti-albumen antibodies are primarily of the IgG4 type.

### Example 3

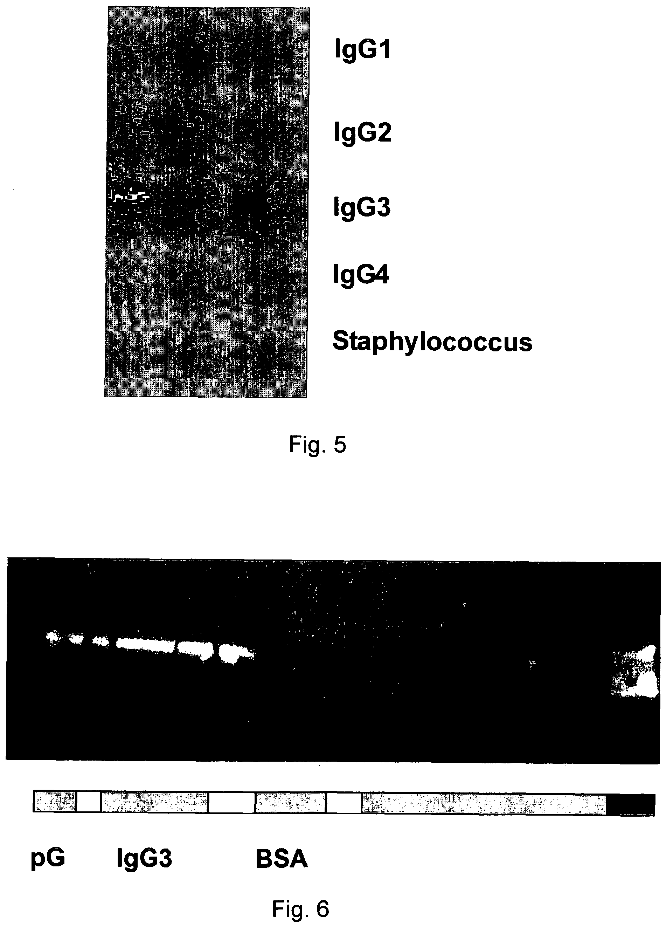

### Use of primary monocytes from human peripheral blood (Figure 5)

Human peripheral blood mononuclear cells were obtained from anticoagulated whole blood by Ficoll-Hypaque density gradient centrifugation. Adherent cells were obtained by incubating mononuclear cells on gelatin-coated Petri-dishes for 1 hour at 37 degrees Celsius, then, after washing the non-adhered cells, the adherent cells were detached by placing the Petri dish on ice in a culture medium containing 20 mM EDTA. The monocytes isolated from peripheral blood were loaded with CellTracker Green (Invitrogen) and were stored at 37°C until use.

Identical concentrations of human IgG subclasses, as well as killed *Staphylococcus aureus* bacteria were printed on 16-pad FAST slides. Human monocytes were applied to the slides under the conditions described in Example 1. The slides were rinsed with phosphate buffer, and 400,000 labelled monocytes in 100 microliters of liquid culture medium were added to one chamber and incubated for 1 hour. Slides were rinsed twice, each time for 10 minutes with liquid culture medium containing no serum to remove non-adhered cells, and were measured using a laser reader after drying.

The binding of the monocytes reflected the biological activities of the printed human antibodies; the highest numbers of them were bound to IgG3 antibodies and negligible numbers of monocytes were bound to other subclasses.

### Example 4

### The effects of anti-human IgG F(ab')₂ treatment on the adhesion of cells to IgG subclasses and to Staphylococcus; and the cells appearing on the scanned chips (Figure 4)

Human IgG subclasses, as well as killed *Staphylococcus* bacteria were printed on nitrocellulose-coated FAST slides. Prior to adding the fluorescently labelled cells, an experiment was performed in order to clarify the role of receptors presumably involved in cell adhesion, in which experiment an attempt was made to inhibit adhesion through the Fc receptors. Printed IgG molecules were coated with anti-human IgG F(ab')₂ to block the adhesion mediated by the Fc receptors. This blocking prevented cell adhesion completely, supporting the notion that the relationship between IgG molecules and their receptors plays a key role in cell adhesion. These results reflect 3 independent experiments and two technical replicates per experiment.

### Example 5 (Figure 6)

### Creating a one-dimensional array in a capillary

A one-dimensional array was created on the internal surface of a 10-microliter glass capillary. For this purpose, washing with ethanol was followed by a pretreatment with 4% 3-aminopropyl-triethoxi-silane, washing steps with acetone and water and a drying step, and 2% glutaric aldehyde was used to treat the internal surface of the capillary. After another washing step with water, antigens were filled in the indicated order into the capillary with air bubbles inbetween them. This step ensures that antigens attach to the internal surface of the capillary in a successive order thereby creating a one-dimensional array. After incubation, the capillary was rinsed with phosphate buffer. Next, fluorescently labelled U937 cells were loaded in the capillary and incubated for 1 hour at 37°C. After removal of the non-adhered cells by washing, the cells were visualised under blue light.

As is visible, cells did not adhere to BSA, only to the superantigen called protein G (pG) and to IgG3. In conclusion, cells used according to the present disclosure will only adhere to the features of a one-dimensional array created in a capillary if the features comprise biologically active substances.

### Example 6

### Assessment of the applicability of U937 cells on a microarray (chip) carrying autoimmune antigens

To assess the applicability of the cell line on complex SLE chips, protein chips were developed with the serum of healthy controls and autoimmune patients, using the U937 cell line and antibodies.

The substances listed in Table 2 were printed on 2-subarray Whatman slides. The chips were washed with PBS on a shaker, once rapidly (1 ml) and then three times for 5-5 minutes (800 µl). A water-air pump was used to remove the liquid from the chambers, and the membranes thus hydrated were incubated for 1 hour at 37°C on a shaker, in a wet chamber using serums diluted in 350 µl Ca-Mg-VBS-BSA buffer in a ratio of 1:5 per subarray. After this incubation step, the chambers were first washed with PBS+Tween and then washed with PBS once rapidly and for 2x10 minutes (only PBS-Tween washing was used when chips were developed with antibodies). PBS was left on the chips until cells were added. 700 µl of a cell suspension containing 10⁷ cells/ml or 700 µl BSA-PBS-Tween containing a 1:2,500 dilution of anti-human IgG-DyL649 (Jackson) were added to each chamber, and were incubated for 30 minutes at 37°C under the same conditions as for the incubation with serum. Next, the chips were washed with PBS once rapidly and for 2×5 minutes, and after removing the frame, they were again washed with PBS once rapidly and for 2×5 minutes in a plastic box. The chips were then dried under a hood and were scanned (settings: Cytotracker-FITC: 488 nm, standard blue filter, PMT 350, 10% amplification, DyL649 : 635 nm, standard red filter, PMT 300, 10% amplification).

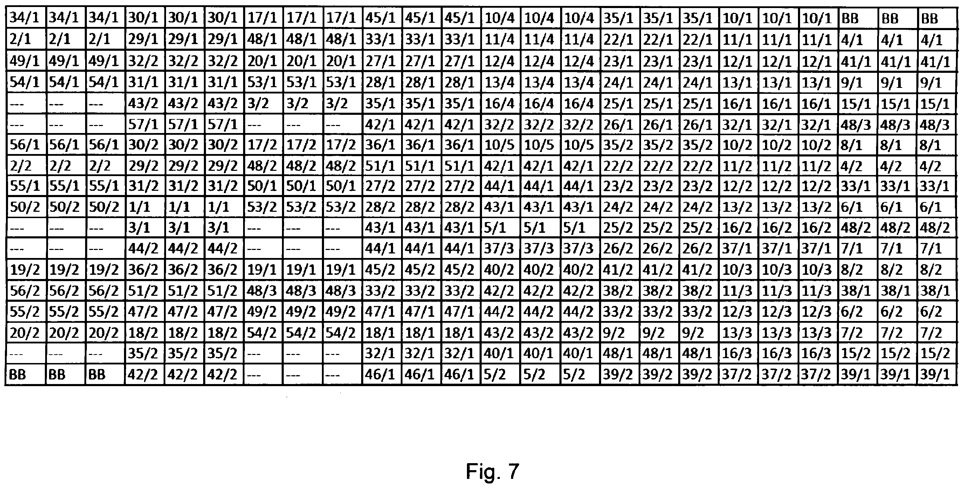

In the representative figures (Figures 7 to 11), the signals of the antibodies and cells adhering to the antigens and the immune profiles thus generated for the individual treatments can be compared.

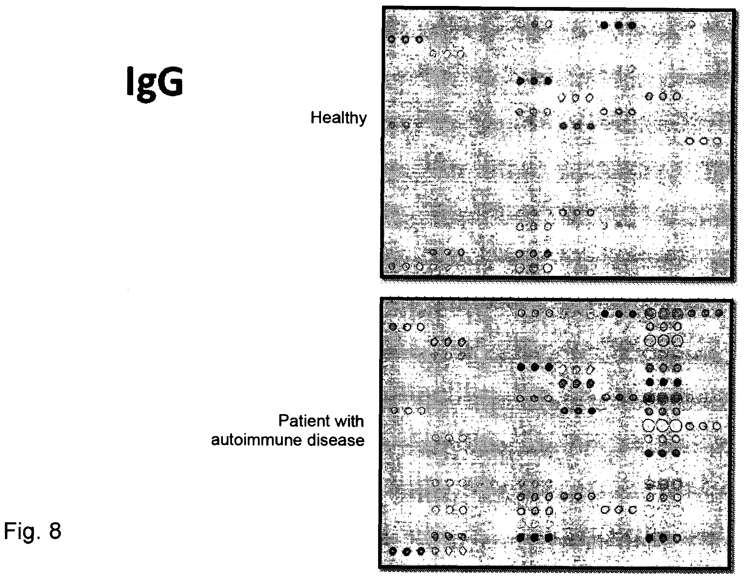

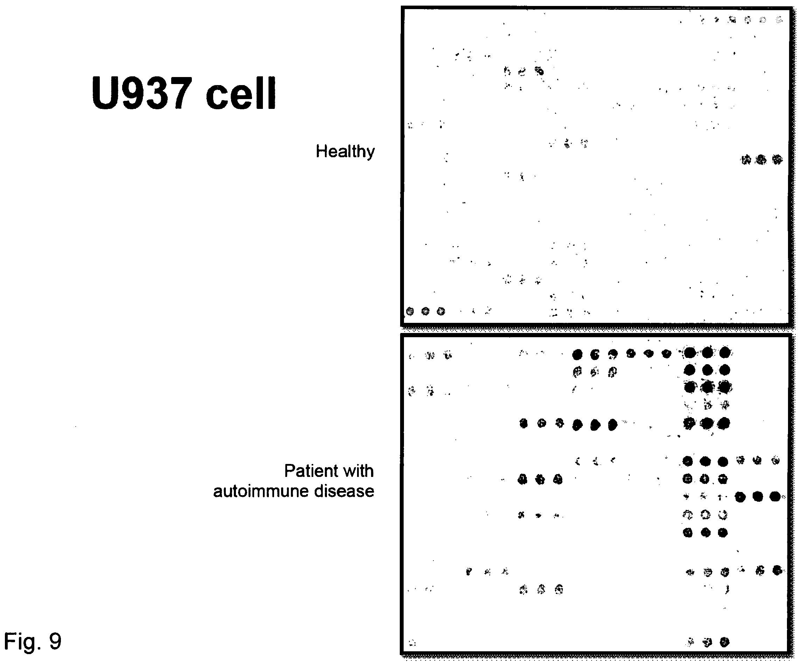

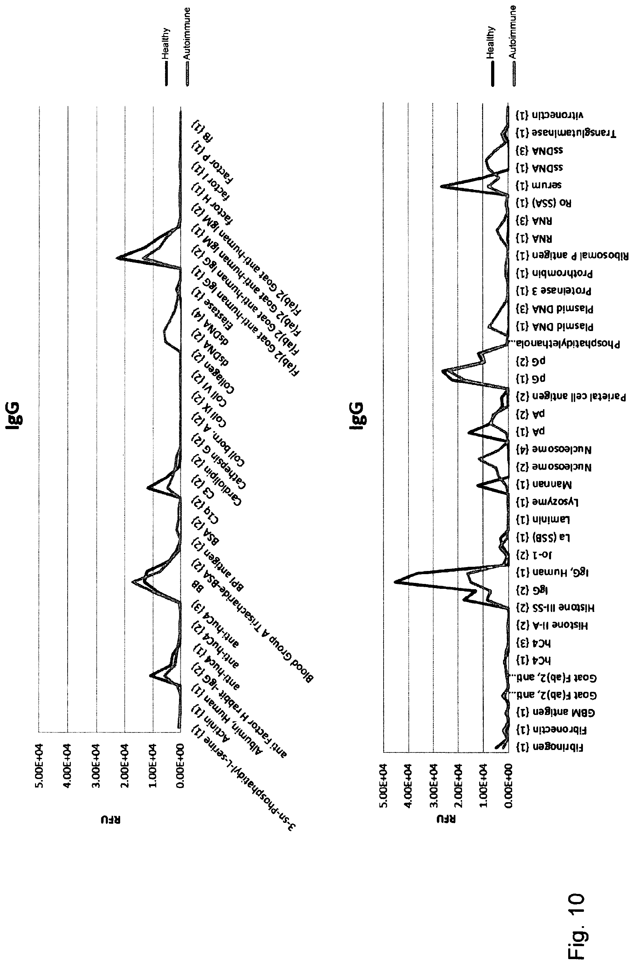

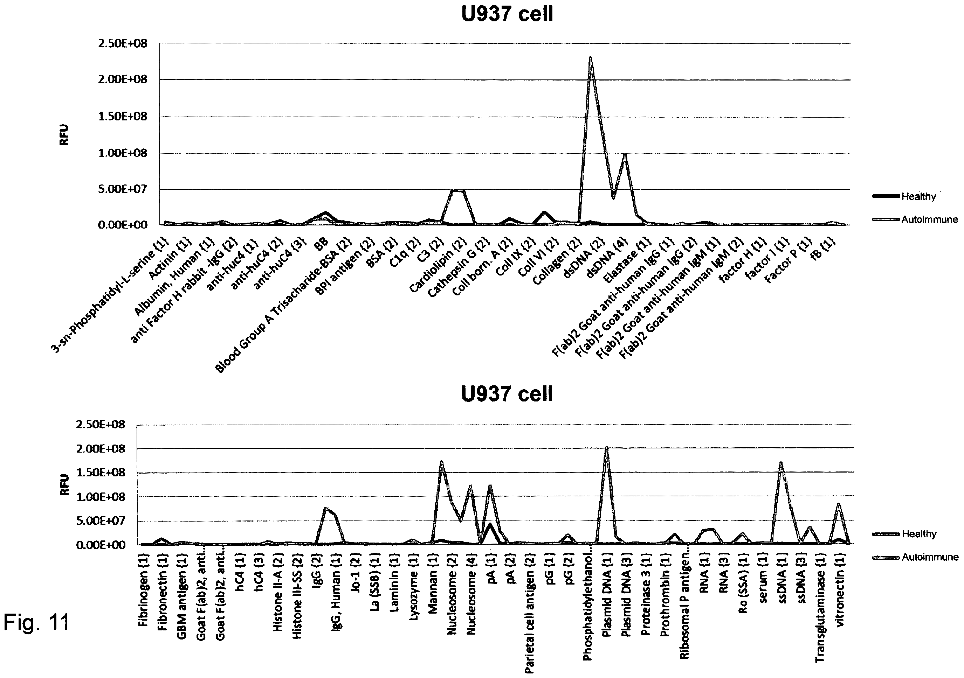

The 58 substances listed in Table 1 were printed on the slides in an array format as shown in Figure 7, some in multiple dilutions (in Figure 7, these are indicated by the indices following the slash). Each substance is present in at least three spots beside each other, as technical replicates. Figure 8 shows the pattern, i.e., antibody profile created by detection antibodies recognising IgGs. It demonstrates that more than one antibodies reacting with the features of the antigen array were present in the autoimmune patient. Figure 9 shows the adhesion of U937 monocytoid cells on the antigen arrays contacted with serums from healthy individual and autoimmune patient, respectively. In the case of the serum from the autoimmune patient, increased reactivity towards the features of the antigen array is clearly visible as indicated by the increased number of cells adhering to the individual features. Figure 10 is the graphical representation of the data derived from the picture in Figure 8. Fluorescence intensities (RFU, relative fluorescence units) corresponding to each antigen are shown and different colours were used for the curves of the healthy subjects and for those of the autoimmune patient. Figure 11 is the graphical representation of the pictures in Figure 9, according to the above. In addition, the curves reflecting the reactivity of the healthy subject and that of the autoimmune subject towards the antigen array are running differently when detection is carried out by detection antibodies (Figure 10) and by cells (Figure 11), respectively. However, cellular detection shows more definite differences between the two subjects tested.

**Table 2**

| | material | description | c (mg/mL) | dilution |
|---|---|---|---|---|
| 1 | Hemocyanin from keyhole limpet (*Megathura crenulata*) | KLH | 1.0 | |
| 2 | Human serum | Serum | [1:50] | 1:5 |
| 3 | BSA (bovine serum albumin) | BSA | 1.0 | 1:5 |
| 4 | 3-sn-Phosphatidyl-ethanolamin from lamb brain, Type II-S | Phosphatidyl-ethanolamin | 1.0 | 1:5 |
| 5 | 3-sn-Phosphatidyl-L-serine | 3-sn-Phosphatidyl-serine | 1.0 | 1:5 |
| 6 | BPI (baktericidal/permeability-increasing protein) antigen, from human neutrophil granulocytes | BPI antigen | 0.3 | 1:5 |
| 7 | C1q, human | C1q | 1.0 | 1:5 |
| 8 | Cardiolipin, solution, from bovine heart | Cardiolipin | 1.0 | 1:5 |
| 9 | Cathepsin G, from human neutrophil granulocytes | Cathepsin G | 1.0 | 1:5 |
| 10 | Double-stranded(ds)DNA, calf thymus DNA | dsDNA | 1.0 | 1:5 |
| 11 | Single-stranded(ds)DNA, calf thymus DNA | ssDNA | 1.0 | 1:5 |
| 12 | Plasmid DNA | Plasmid DNA | 1.0 | 1:5 |
| 13 | RNA | RNA | 1.0 | 1:5 |
| 15 | Actinin | Actinin | 1.0 | 1:5 |
| 16 | Nucleosome, human, from cell line | Nucleosome | 0.6 | 1:5 |
| 17 | Elastase, from human neutrophil granulocytes | Elastase | 1.0 | 1:5 |
| 18 | Fibrinogen, Fraction I, Type I: from human plasma | Fibrinogen | 2.0 | 1:5 |
| 19 | Fibronectin, from bovine plasma | Fibronectin | 1.0 | 1:5 |
| 20 | GBM antigen, bovine kidney | GBM antigen | 0.5 | 1:5 |
| 22 | Histone, from calf thymus, Type II-A | Histone IIA | 1 | 1:5 |
| 23 | Jo-1 antigen, calf thymus | Jo-1 | 0.6 | 1:5 |
| 24 | La (SSB) antigen, calf thymus | La(SSB) | 0.8 | 1:5 |
| 25 | Lysozyme, from chicken albumen (muramidase) | Lysozyme | 1.0 | 1:5 |
| 26 | Parietal cell antigen, porcine | Parietal cell antigen | 0.6 | 1:5 |
| 27 | Proteinase 3 | Proteinase 3 | 0.8 | 1:5 |
| 28 | Prothrombin, from human plasma | Prothrombin | 0.8 | 1:5 |
| 29 | Ribosomal P antigen, calf thymus | Ribosomal P antigen | 0.5 | 1:5 |
| 30 | Ro (SSA) antigen, calf thymus | Ro(SSA) | 0.3 | 1:5 |
| 31 | Transglutaminase, rec. human | Transglutaminase | 0.4 | 1:5 |
| 32 | Protein G, from *Streptococcus sp.* | pG | 0.5 | 1:5 |
| 33 | Protein A, from *Streptococcus aureus,* soluble | pA | 1.0 | 1:5 |
| 34 | Albumin, human | Albumin, human | 1.0 | |
| 35 | IgG, human | IgG, human | 0.5 | 1:5 |
| 36 | C3, human | C3 | 1.0 | 1:5 |
| 37 | Purified human C4 complement protein | hC4 | 1.0 | 1:5 |
| 38 | Factor P, human | Factor P | 0.5 | 1:5 |
| 39 | Factor H, human | Factor H | 1.0 | 1:5 |
| 40 | Factor I, human | Factor I | 1.0 | 1:5 |
| 41 | Factor B, human | fB | 0.7 | 1:5 |
| 42 | F(ab)₂ goat anti-human IgG | F(ab)₂ goat anti-human IgG | 1.0 | 1:5 |
| 43 | F(ab)₂ goat anti-human IgM | F(ab)₂ goat anti-human IgM | 1.0 | 1:5 |
| 44 | Goat F(ab)₂, anti-human C3 | F(ab)₂ goat anti | 1.0 | 1:5 |
| 45 | Anti-Factor H rabbit IgG | Anti-factor H rabbit IgG | 2.0 | 1:5 |
| 46 | Mannan (*Saccharomyces cerevisiae*) | Mannan | 10.0 | |
| 47 | Blood group A trisaccharide-BSA, 10-atom spacer | Blood group A trisaccharide BSA | 1.0 | 1:5 |
| 48 | Anti-huC4 | Anti-huC4 | 2.0 | 1:5 |
| 49 | Histone III-SS | Histone III-S | 1.0 | 1:5 |
| 50 | Laminin | Laminin | 0.5 | 1:5 |
| 51 | Vitronectin | Vitronectin | 0.4 | 1:5 |
| 53 | Collagen VI | Coll VI | 1.0 | 1:5 |
| 54 | Collagen, Born. A | Coll Born A | 1.0 | 1:5 |
| 55 | Collagen IX | Coll IX | 1.0 | 1:5 |
| 56 | Collagen | Collagen | 1.0 | 1:5 |
| 57 | PBS-azid | PBS | | |
| BB | BB+20x 488; 20x cy5 | BB | | |

### Example 7

### Liquid flow changes in microcapillaries treated with various IgG subclasses, in the case of using whole blood

Background: The myeloid cells bind to IgG3, and not to IgG2 on the microarrays. By applying these two IgGs of different functions, it can be determined whether in arrays made from microcapillaries, cell adhesion changes the liquid flow rate or not.

Implementation:
1) Capillaries:
   a. Vitrotubes, round capillary, Cat No.: CV1525, 0.15IDx0.25 OD, Lot Code: 3140Z1 (www.vitrocom.com)
   b. Vitrotubes, square capillary, Cat No.: 8505-050, 0.050IDx0.025mm Wall, Lot Code: 0990C1 (www.vitrocom.com)
2) Three capillaries of different assemblies are made (A, B, C), two from each for the parallel measurements (I and II).
   A) Capillary without inner constriction
      a. treatment with 0.25M EDTA for 30 minutes, drying
   B) A capillary furnished with an inner constricting capillary and treated with nitrocellulose and IgG2
      a. treatment with 0.25M EDTA for 30 minutes, drying
      b. 0.2% Nitrocellulose solution (ethanol: diethyl-ether, 1:1), 5 min, drying
      c. Immersion into 0.5mg/ml hIgG2 solution, for 20 minutes, drying (human IgG2, Sigma, I 5404-1MG, 058K6116)
      d. trimming 2cm at the edge, which will then be used for constriction
      e. inserting the constricting element into the outer capillary
   C) Capillary treated with nitrocellulose and IgG3, furnished with an inner constricting capillary
      a. treatment with 0.25M EDTA for 30 minutes, drying
      b. 0.2% nitrocellulose solution (ethanol: diethyl-ether, 1:1), 5 min., drying
      c. Immersion into 0.5mg/ml hIgG3 solution, for 20 minutes, drying (human IgG3, Sigma I 5654-1MG, 059K6007)
      d. trimming 2cm at the edge, which will then be used for constriction
      e. inserting the constricting element into the outer capillary
3) Put one drop of blood drawn from the fingertip into the narrowed inlet of the capillaries

### results:

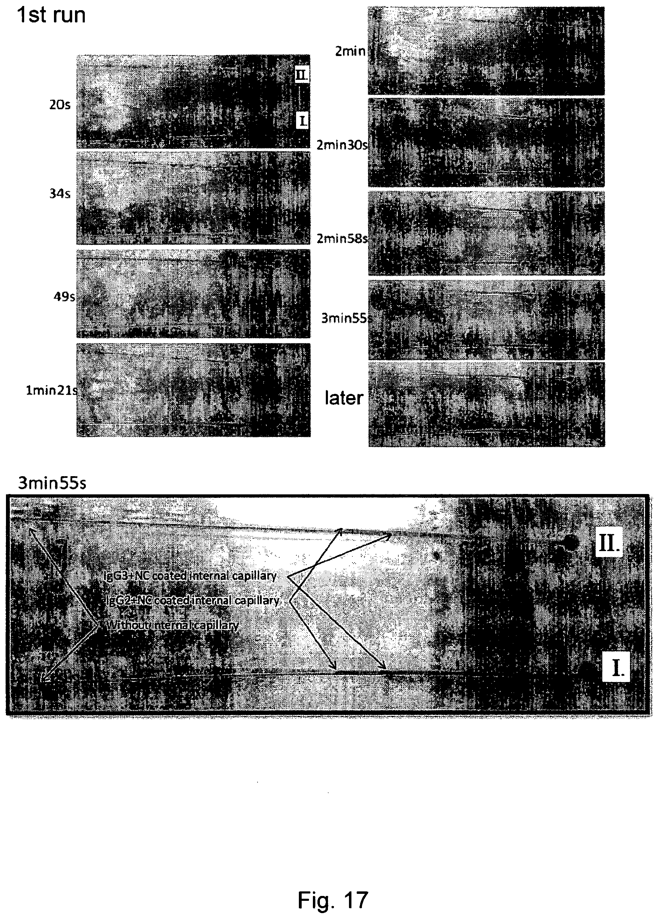

The results are shown in Figure 17. The running of sample 'B' started 7 seconds later than the running of sample 'A'. The times given next to the figures apply to sample 'A', the 's' stands for seconds and 'min' for minutes.

The results show that
- blood runs quickly along a capillary which does not have constriction, and
- blood proceeds faster in a capillary containing a constricting capillary treated with IgG2 than in a capillary comprising a constricting capillary treated with IgG3

### Example 8

HBsAg test in capillaries

### Purpose of the experiment

Detecting an antigen-specific antibody response in a delayed flow test, in capillaries, using hepatitis B antigen

### Materials

Slide: VWR ECN 631-1553
Embedding material for outer capillary: PDMS, Dow Corning, Sylgard 184
Outer capillary: Vitrocom CV1525
Inner capillary: Vitrocom 8505-050
Hepatitis antigen (HBsAg): HBV Surface Antigen adw protein; Biorbyt, orb82659
Bovine albumin: Sigma A-3059
Silane: Sigma-Aldrich, A3648

### Preparation of outer capillaries

Six capillaries are arranged accurately side by side, leaving 5mm protruding ends for forming the inlet for the blood drops, and then 1ml PDMS solution is layered on the slide, uniformly distributing same on a horizontal surface, followed by baking at 80 °C for one hour.

### Preparation of inner capillaries

One end of each capillary is sealed by gas flame.

The open end is immersed into 2% silane/acetone solution, dried, then immersed in water and dried again.

The open end is placed into HBsAg solution of 0.2mg/ml concentration at 3cm depth (plastic sheath of medical syringe) and in the meantime negative control capillaries are placed into 5% BSA solution in the same way, stored at 37°C for one hour, to form the antigen coating.

The unbound antigen is removed by immersing the capillary into the following solutions consecutively: PBS, PBS, 5% BSA solution in PBS, PBS, water. The capillary is dried.

### Forming the capillary trap and reader

The open antigen coated ends of inner capillaries are inserted 2cm deep into the outer capillaries fixed to the slides. 3 inner capillaries coated with HBsAg antigen provide the measuring system, and 3 capillaries coated with BSA provide the reference negative control. The capillaries are fixed to one another by an adhesive tape, creating an inlet gate for the blood drop.

### Measurement

Capillary blood drawn from fingertip is supplemented to 20mM EDTA concentration to prevent coagulation. 25 microliters of blood is pipetted into the inlet gate, uniformly distributing the blood on the six capillaries. The blood flow is tracked and registered in time.

The structure of HBsAg test is shown in Figure 18. In Figure 18 inner capillary channels 30 and 32 are divided into two groups, according to the Figure, in outer capillary channels 31 arranged on slide 34. In the present embodiment the outer surface of the inner capillary channels 30 is coated with HBsAg antigen, while that of the inner capillary channels 32 is coated with BSA. In Figure 18 spot 29 indicates the place where the sample, i.e. preferably blood drop has to be dripped.

### Results

The distance covered by the blood flowing in the capillaries is shown in Table 3, measured from the end of the trap, in mm.

**Table 3**

| *antigen* | *5 minutes* | *10 minutes* | *15 minutes* |
|---|---|---|---|
| HBsAg-1 | 11 | 13 | 16 |
| HBsAg-2 | 6 | 24 | 32 |
| HBsAg-3 | 2 | 14 | 16 |
| **Average - HBsAg** | **6,3** | **17,0** | **21,3** |
| BSA-1 | 15 | 49 | 71 |
| BSA-2 | 13 | 38 | 72 |
| BSA-3 | 11 | 31 | 36 |
| **Average - BSA** | **13,0** | **39,3** | **59,6** |

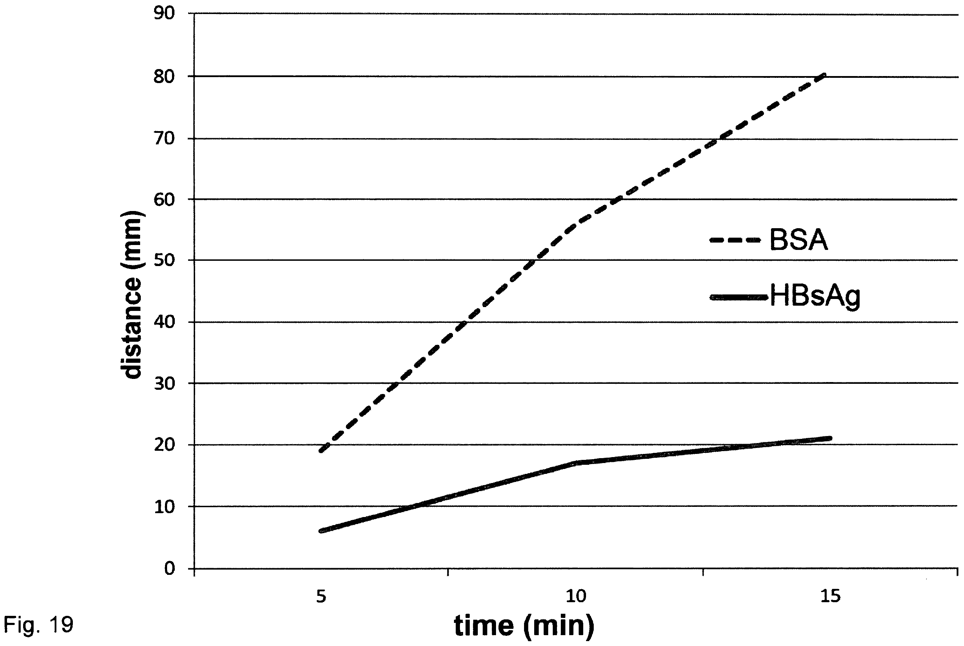

The chart of the results of the experiment performed is shown in Figure 19.

### Evaluation

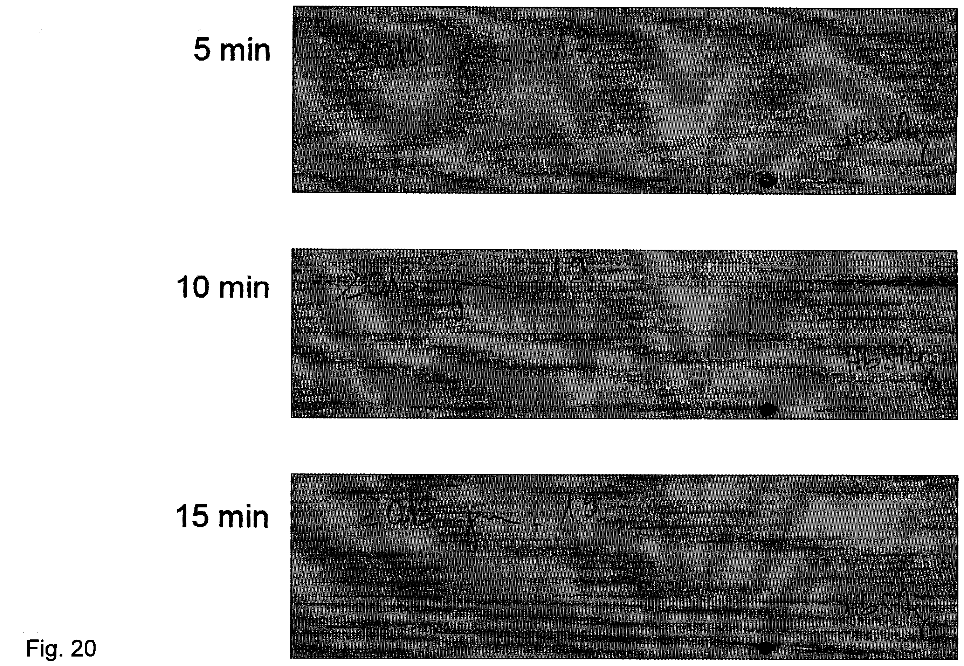

In the capillaries coated with HBsAg antigen, the blood flow induced (moved) by capillary forces significantly slows down, when the blood of a subject having known HBsAg reactivity is used. The result of the experiment is shown in Figure 20.

### Example 9

The effect of human IgG3, IgG2 and serum albumin on the blood flow in microcapillaries (Figure 14)

### Purpose

The myeloid cells adhere to IgG3, but not to IgG2 and albumin, thus the experiment serves for proving that the blood flow rate changes as a result of cell adhesion.

### Method

Microcapillaries:
a. Vitrotubes, round capillary, Cat No.: CV1525, 0.15IDx0.25OD, Lot Code: 3140Z1 (www.vitrocom.com)
b. Vitrotubes, square capillary, Cat No.: 8505-050, 0.050IDx0.025mm Wall, Lot Code: 0990C1 (www.vitrocom.com)

The experiment was carried out as described in Example 7, with the difference that beyond IgG3 and IgG2, albumin was also used for coating an inner capillary, and that an empty outer capillary was not used.

### Conclusion

An inner capillary coated with IgG3 slows down the blood flow in the outer capillary.

The result of the experiment is shown in Figure 14.

### Example 10

### Purpose

Measuring the adhesion of neutrophil granulocytes on an antigen array

### Method

1) Antigen arrays comprising IgG subclasses, PBS and anti-human IgG antibodies (ahIgG) are made on a nitrocellulose surface
2) Whole blood or lysed blood freed from red blood cells is added to rehydrated arrays, the arrays are incubated for 1 hour at 37°C and the adherent cells are labelled by anti-human CD66abce-FITC (Dako Cat.:F7112 Lot 00034553) antibodies.

After washing, the arrays were scanned by a laser reader.

### Result

The neutrophil granulocytes adhere (bind) to the subclasses IgG3 and IgG1.

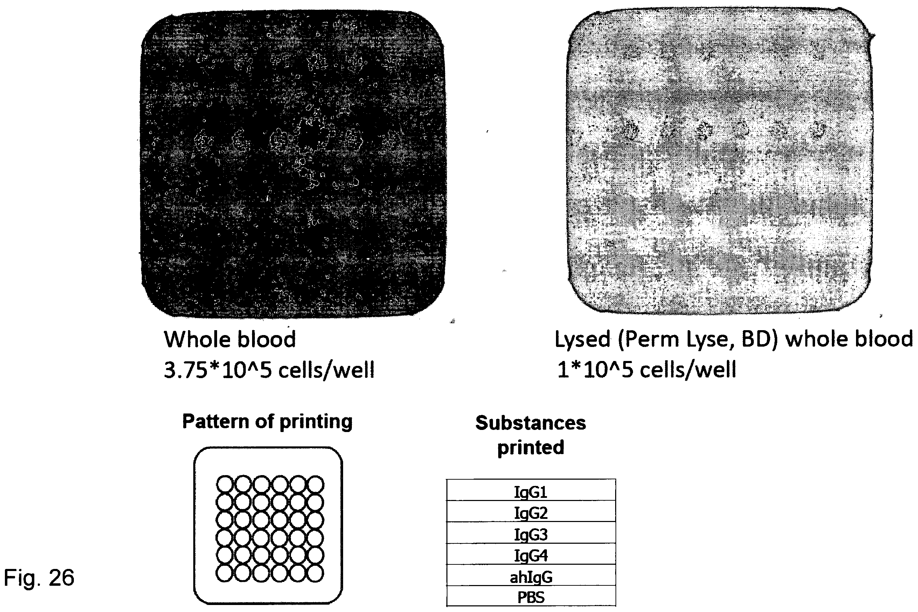

The experimental result is shown in Figure 26.

### Example 11

Retardation (slowing-down) of blood flow by surface-bound antibody in a microfluidic system (capillary channel system)

### Purpose:

Two microfluidic channels (capillary channels) differing in their coatings are compared to prove retardation of blood flow by an antibody that interacts with Fc receptors on neutrophil granulocytes

### Principle:

Two materials are compared in the test: bovine serum albumin (BSA) is inert with respect to FcR binding, serving as a negative control antigen, human IgG3 antibody is highly active at binding FcRs of cells, serving as positive control. Neutrophil granulocytes in the blood are expected to bind to IgG3, adhere to the channel wall, constrict passage through the microfluidic channel and thereby slow down the flow of blood.

### Generation of the PDMS microfluidic structure

The macroscopic design for the microfluidic operational unit is shown in Figure 27, and the microscopic design thereof in Figure 28.

Experiments were conducted with channels that were 15 µm high. The devices were generated using soft lithography. PDMS (Sylgard 184 PDMS prepolymer, Dow Corning, Midland, Ml) was cast on the silicon disc master on which the positive relief was formed by SU8 photoresist. The PDMS was then cured at 80 °C in a convection oven for 60 minutes. The cured PDMS was peeled off the master, cut to appropriate size to fit the Nexterion slides.

### Preparation of the surface serving as a basis for the microfluidic system

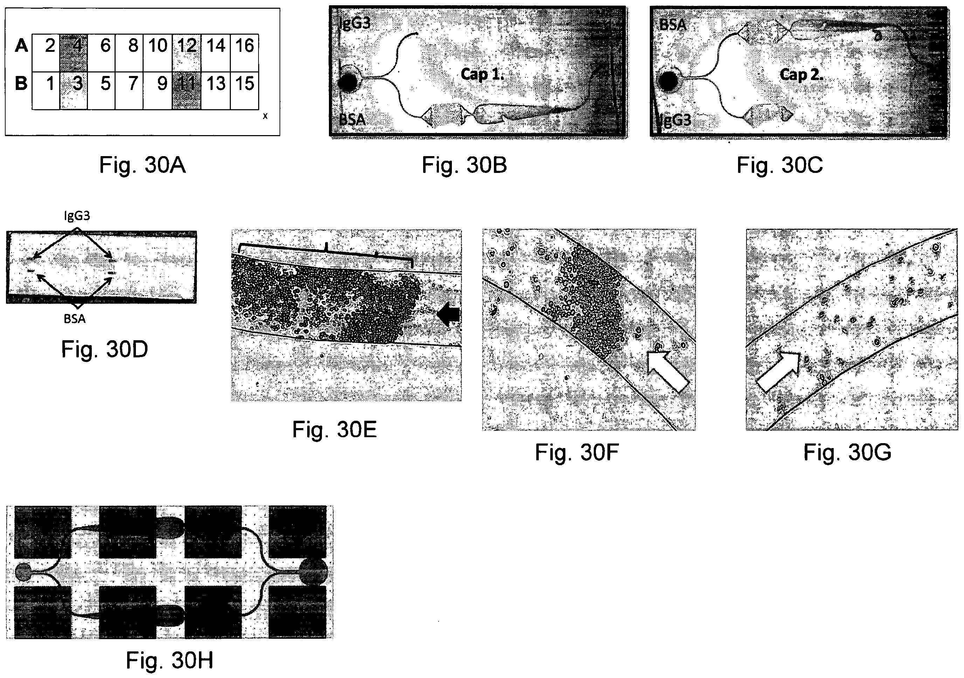

1) Nexterion 1 pad slide is inserted into ProPlate 16 well chamber
Slide: SCHOTT Nexterion Slide H; Hydrogel coated;
CatNo: 1070936; LotNo:4-2882
Arrangement is shown in Figure 30A. Only chambers marked with numbers 3, 4, 11 and 12 (shown in figure 30A with median-grey and dark-grey colouring) were treated, the others were left empty. First the chamber was rinsed with water then air pressure was used to remove liquid.
2) Chambers were filled with 100µl of solution, and incubated at 37°C for 1h
Sub-arrays 4&11: 50µg/ml IgG3
Sub-arrays 3&12: 50µg/ml BSA
IgG3 was first dialyzed against PBS, the stock concentration was 0.78mg/ml

| Manufacturer | Cat.No. | Lot.No. | Full name |
|---|---|---|---|
| Sigma | A3059-100G | 100M1767V | Albumin, bovine serum, fraction V, |
| | | | Approx. 99% Protease-free, Essentially gamma-globulin free |
| Sigma | I5654-1MG | 091M6211 | HUMAN IGG3, KAPPA, FROM |
| | | | HUMAN PLASMA |

3) Washing 2x5min PBS (in 16 pad chambers) 100µl/each chamber
4) Disassembly of chambers
5) Blocking: incubation in 100mM Tris (pH8) at room temperature for 1h
6) Washing 3x5min PBS
7) Rinsing with MilliQ water then centrifugation to remove liquid

### Mounting of the microfluidic system (capillary channel system)

1) capillary system:
i. PDMS block (14.2µm height) is air plasma treated (6s, 800W microwave, vacuum<1mBar).

2) Blood (collected in two different ways):
i. 5x diluted-blood
   1. Blood was collected into eppendorf tube that already contained PBS until 5x dilution was reached
ii. Citrate-blood
   1. Blood was collected (drop by drop) into eppendorf tube that already contained 3.8% Na-citrate solution (ratio of blood and 3.8% sodium-citrate solution: 9:1)

3) Capillary channel system was prepared on the day of the measurement and was incubated for 2h at room temperature before 3µl blood was added to it.

| Slide | Part I | Part II |
|---|---|---|
| 10067683 | 5xdiluted-blood (Cap1) | Citrate-blood (Cap2) |

Capillary channel system 1 (Cap1)
   - The flow over the BSA-coated chamber was faster, it was less than 3min
      The result obtained with Capillary channel system 1 is shown in Figure 30B.
Capillary channel system 2 (Cap2):
   - The flow over the BSA-coated chamber was faster, it was less than 3min
      The result obtained with Capillary channel system 2 is shown in Figure 30C.

Blood flow reaching the junction of the two channels effectively stops liquid flow in the other channel.

### Example 12

Testing human neutrophil binding to BSA-IgG3-coated PDMS device

### Method:

1) Materials:
Materials of concentration of 0.5mg/ml were used for coating, dilution was carried out in PBS.
Note: The IgG3 was dialyzed against PBS

| | Manufacturer | Cat.No. | Full name |
|---|---|---|---|
| BSA | Sigma | A3059-100G | Albumin, bovine serum |
| human IgG3 | Sigma | | HUMAN IGG3, KAPPA, FROM |
| | | I5654-1MG | HUMAN PLASMA |

2) Chip map:
Nexterion slides were coated (SCHOTT Nexterion Slide H; Hydrogel coated; CatNo: 1070936; LotNo: 4-2882). According to Figure 30D, to the areas indicated by arrow 3-3µl of BSA solution and IgG3 solution, respectively, were added by pipette.
3) Protein immobilization: Incubation at 37°C for 30min
4) Blocking: Incubation in 100mM Tris (pH8) at room temperature for 1h
5) Washing 3x5min in PBS, rinsing in Milli Q water and centrifuging for 1min to dry
6) PDMS blocks with 14µm height channels were air plasma treated and inserted onto the coated slides. A coated slide is shown in Figure 30H.
7) Incubation in humidified chamber at room temperature for overnight.
8) Neutrophil granulocytes:
a. Neutrophils were isolated on Ficoll gradient from human whole blood.
b. 1.5*10⁷ cell/ml concentration was set in RMPI 10% FCS medium.

9) 3µl of cell suspenion was introduced to the sample gate of the microfluidic device (capillary channel system) and the events were checked under microscope.

### Results:

Human neutrophil granulocytes bind to the IgG3-coated section of the capillary channel. The result observed by microscope is shown in Figure 30E.

### Example 13

U937 BSA-IgG3
Figure 30F: capillary coated with IgG3
Figure 30G: capillary coated with BSA
Testing U937 cell binding to BSA-IgG3 coated PDMS device

### Method:

1) Materials:
Materials of concentration of 0.5mg/ml were used for coating, dilution was carried out in PBS.
NOTE: The IgG3 was dialyzed against PBS

| | Manufacturer | Cat.No. | Full name |
|---|---|---|---|
| BSA | Sigma | A3059-100G | Albumin, bovine serum |
| human | Sigma | | HUMAN IGG3, KAPPA, FROM |
| IgG3 | | I5654-1MG | HUMAN PLASMA |

2) Chip map:
Nexterion slides were coated (SCHOTT Nexterion Slide H; Hydrogel coated; CatNo:1070936; LotNo: 4-2882). According to Figure 30D, to the areas indicated by arrow 3-3µl of BSA solution and IgG3 solution, respectively, were added by pipette.
3) Protein immobilization: Incubation at 37°C for 30min
4) Blocking: Incubation in 100mM Tris (pH8) at room temperature for 1h
5) Washing 3x5min in PBS, rinsing in Milli Q water and centrifuging for 1min to dry
6) PDMS blocks with 14µm height channels were air plasma treated and inserted onto the coated slides. A coated slide is shown schematically in Figure 30H.
7) Incubation in humidified chamber at room temperature for 2h.
8) Preparation of a suspension of a concentration of 5*10⁶ U937 cell/ml in RMPI 10% FCS medium.
9) 3µl of cell suspension was introduced to the sample gate of the microfluidic device (capillary channel system) and the events were checked under microscope.

### Results:

U937 cells bind to the IgG3-coated section of the capillary channel. The results are shown in Figures 30F and 30G. In Figure 30F microscopic image of the section coated by IgG3 of the capillary channel is shown. Large number of U937 cells were bound (adhered) to the section analysed. In Figure 30G microscopic image of the section coated by BSA of the capillary channel is shown.

In the section analysed the U937 cells do not adhere to the extent as on the section coated by IgG3 of the capillary channel.

## Claims

1. A capillary channel system, which comprises
- a measuring channel (106), which measuring channel (106) is a capillary channel comprising
o a trap section (1, 22, 38, 40, 42, 44, 46, 47, 48) having a coating comprising an antigen, the trap section (1, 22, 38, 40, 42, 44, 46, 47, 48) enables the adhesion of cells of a sample,
o a collector section (2, 20) having a volume which is at least as large as that of the trap section (1, 22, 38, 40, 42, 44, 46, 47, 48), and being adapted for receiving sample flowing through the trap section (1, 22, 38, 40, 42, 44, 46, 47, 48), and
∘ a reader section (3, 18) adjacent to the collector section (2, 20),
wherein the collector section (2, 20) is located in the part between the trap section (1, 22, 38, 40, 42, 44, 46, 47, 48) and the reader section (3, 18) of the capillary channel, and
- a negative control channel (10, 104) having a structural configuration identical with that of the measuring channel (106) and being provided with a coating which differs from that of the measuring channel (106) or being formed without coating.

2. The capillary channel system according to claim 1, **characterised in that** the trap section (1, 22, 38, 40, 42, 44, 46, 47, 48) comprises at least one passage having a traversable cross-sectional area with a characteristic size of 1 to 20 micrometer, preferably 10 micrometer or less.

3. The capillary channel system according to claim 1 or 2, **characterised in that** the trap section (1, 22, 38, 40, 42, 44, 46, 47, 48) comprises a constricting element, and the traversable cross-sectional area of the at least one passage is formed by means of the at least one constricting element.

4. The capillary channel system according to any of claims 1 to 3, **characterised in that** the reader section (3, 18) has a transparent wall.

5. The capillary channel system according to any of claims 1 to 4, **characterised in that** the negative control channel (10, 104) which - in case of flowing the same sample - in its part downstream from the trap section (1, 22, 38, 40, 42, 44, 46, 47, 48) provides identical or higher flow rate than that in the measuring channel.

6. The capillary channel system according to any of claims 1 to 5, **characterised by** comprising a positive control channel (16) having a structural configuration identical with that of the measuring channel (106) and comprising a coating which differs from that of the measuring channel, and - in case of flowing the same sample - in its part downstream from the trap section (1, 22, 38, 40, 42, 44, 46, 47, 48) providing identical or lower flow rate than that in the measuring channel.

7. The capillary channel system according to any of claims 1 to 6, **characterised by** comprising more than one measuring channels of identical or different structural configurations.

8. The capillary channel system according to any of claims 1 to 7, **characterised by** comprising more than one negative control channels of identical or different structural configurations.

9. The capillary channel system according to any of claims 1 to 8, **characterised by** comprising more than one positive control channels of identical or different structural configurations.

10. The capillary channel system according to any of claims 1 to 6, **characterised by** comprising a single measuring channel (106) and a single negative control channel (104), and the end points of the measuring channel and the negative control channel (104) are connected to a common draining capillary channel.

11. A method for the analysis of immunoreactivity comprising introducing a sample into a capillary channel system which comprises
- a measuring channel (106), which measuring channel (106) is a capillary channel comprising
∘ a trap section (1, 22, 38, 40, 42, 44, 46, 47, 48) having a coating comprising an antigen, the trap section (1, 22, 38, 40, 42, 44, 46, 47, 48) enables the adhesion of cells of a sample,
∘ a collector section (2, 20) having a volume which is at least as large as that of the trap section (1, 22, 38, 40, 42, 44, 46, 47, 48), and being adapted for receiving sample flowing through the trap section (1, 22, 38, 40, 42, 44, 46, 47, 48), and
∘ a reader section (3, 18) adjacent to the collector section (2, 20),
wherein the collector section (2, 20) is located in the part between the trap section (1, 22, 38, 40, 42, 44, 46, 47, 48) and the reader section (3, 18) of the capillary channel, and
- a negative control channel (10, 104) having a structural configuration identical with that of the measuring channel (106) and being provided with a coating which differs from that of the measuring channel (106) or being formed without coating, and
after the liquid front of the sample in the negative control channel having reached the reader section, observing or measuring on the reader sections of the individual channels a feature of the flow of the sample, preferably the flow rate of the sample or a quantity proportional thereto.

12. The method according to claim 11, wherein the analysis of immunoreactivity is aimed at the diagnosis of a condition or disorder accompanied by the presence of one or more specific antibodies, and the trap section of at least one measuring channel comprises an antigen capable to bind to at least one of said specific antibodies, and the method optionally further comprises comparing the flow features observed or measured in the individual channels, wherein the flow features are preferably the flow rates or quantities proportional thereto.

13. The capillary channel system or method according to any of the preceding claims, wherein the one or more antigens are selected from those listed in Table 1.

14. The capillary channel system or method according to any of the preceding claims, wherein the sample is whole blood.

15. Microfluidic device comprising a capillary channel system according to any of claims 1 to 10, wherein the capillary channel system is formed in the material or materials constituting the device.

16. Use of the capillary channel system according to any of claims 1 to 10, or the microfluidic device according to claim 15 as rapid diagnostic device for point-of-care serological tests.

## Patentansprüche

1. Kapillarkanalsystem, aufweisend
- einen Messkanal (106), wobei der Messkanal (106) ein Kapillarkanal ist, aufweisend
∘ einen Fallenbereich (1, 22, 38, 40, 42, 44, 46, 47, 48) mit einer Beschichtung, die ein Antigen aufweist, wobei der Fallenbereich (1, 22, 38, 40, 42, 44, 46, 47, 48) die Adhäsion von Zellen einer Probe ermöglicht,
∘ einen Sammelbereich (2, 20) mit einem Volumen, welches zumindest so groß ist wie das des Fallenbereiches (1, 22, 38, 40, 42, 44, 46, 47, 48), und angepasst ist zur Aufnahme einer Probe, welche durch den Fallenbereich (1, 22, 38, 40, 42, 44, 46, 47, 48) fließt, und
∘ einen Lesebereich (3, 18) angrenzend an den Sammelbereich (2, 20),
wobei der Sammelbereich (2, 20) in dem Teil zwischen dem Fallenbereich (1, 22, 38, 40, 42, 44, 46, 47, 48) und dem Lesebereich (3, 18) des Kapillarkanals angeordnet ist, und
- einen Negativkontrollkanal (10, 104) mit einer strukturellen Konfiguration, die identisch mit der des Messkanals (106) ist, und der mit einer Beschichtung bereitgestellt wird, die sich von der des Messkanals (106) unterscheidet oder ohne Beschichtung gebildet wird.

2. Kapillarkanalsystem nach Anspruch 1, **dadurch gekennzeichnet, dass** der Fallenbereich (1, 22, 38, 40, 42, 44, 46, 47, 48) zumindest einen Durchlass aufweist, mit einer passierbaren Querschnittsfläche mit einer charakteristischen Größe von 1 bis 20 Mikrometern, vorzugsweise 10 Mikrometern oder weniger.

3. Kapillarkanalsystem nach Anspruch 1 oder 2, **dadurch gekennzeichnet, dass** der Fallenbereich (1, 22, 38, 40, 42, 44, 46, 47, 48) ein einengendes Element aufweist, und dass die passierbare Querschnittsfläche des zumindest einen Durchlasses mittels des zumindest einen einengenden Elementes gebildet ist.

4. Kapillarkanalsystem nach einem der Ansprüche 1 bis 3, **dadurch gekennzeichnet, dass** der Lesebereich (3, 18) eine transparente Wandung hat.

5. Kapillarkanalsystem nach einem der Ansprüche 1 bis 4, **dadurch gekennzeichnet, dass** der Negativkontrollkanal (10, 104) - im Falle des Fließens der gleichen Probe - in seinem Teil stromabwärts des Fallenbereichs (1, 22, 38, 40, 42, 44, 46, 47, 48) eine identische oder höhere Fließrate als die in dem Messkanal bereitstellt.

6. Kapillarkanalsystem nach einem der Ansprüche 1 bis 5, **dadurch gekennzeichnet, dass** es einen Positivkontrollkanal (16) aufweist, mit einer strukturellen Konfiguration, die identisch mit der des Messkanals (106) ist, und der eine Beschichtung aufweist, die sich von der des Messkanals unterscheidet, und der - im Falle des Fließens der gleichen Probe - in seinem Teil stromabwärts des Fallenbereichs (1, 22, 38, 40, 42, 44, 46, 47, 48) eine identische oder niedrigere Fließrate als die in dem Messkanal bereitstellt.

7. Kapillarkanalsystem nach einem der Ansprüche 1 bis 6, **dadurch gekennzeichnet, dass** es mehr als einen Messkanal von identischer oder unterschiedlicher struktureller Konfiguration aufweist.

8. Kapillarkanalsystem nach einem der Ansprüche 1 bis 7, **dadurch gekennzeichnet, dass** es mehr als einen Negativkontrollkanal von identischer oder unterschiedlicher struktureller Konfiguration aufweist.

9. Kapillarkanalsystem nach einem der Ansprüche 1 bis 8, **dadurch gekennzeichnet, dass** es mehr als einen Positivkontrollkanal von identischer oder unterschiedlicher struktureller Konfiguration aufweist.

10. Kapillarkanalsystem nach einem der Ansprüche 1 bis 6, **dadurch gekennzeichnet, dass** es einen einzigen Messkanal (106) und einen einzigen Negativkontrollkanal (104) aufweist, und dass die Endpunkte des Messkanals und des Negativkontrollkanals (104) mit einem gemeinsamen Abflusskapillarkanal verbunden sind.

11. Verfahren zur Analyse der Immunreaktivität, aufweisend Einbringen einer Probe in ein Kapillarkanalsystem aufweisend
- einen Messkanal (106), wobei der Messkanal (106) ein Kapillarkanal ist, aufweisend
∘ einen Fallenbereich (1, 22, 38, 40, 42, 44, 46, 47, 48) mit einer Beschichtung, die ein Antigen aufweist, wobei der Fallenbereich (1, 22, 38, 40, 42, 44, 46, 47, 48) die Adhäsion von Zellen einer Probe ermöglicht,
∘ einen Sammelbereich (2, 20) mit einem Volumen, welches zumindest so groß ist wie das des Fallenbereiches (1, 22, 38, 40, 42, 44, 46, 47, 48), und angepasst ist zur Aufnahme einer Probe, welche durch den Fallenbereich (1, 22, 38, 40, 42, 44, 46, 47, 48) fließt, und
∘ einen Lesebereich (3, 18) angrenzend an den Sammelbereich (2, 20),
wobei der Sammelbereich (2, 20) in dem Teil zwischen dem Fallenbereich (1, 22, 38, 40, 42, 44, 46, 47, 48) und dem Lesebereich (3, 18) des Kapillarkanals angeordnet ist, und
- einen Negativkontrollkanal (10, 104) mit einer strukturellen Konfiguration, die identisch mit der des Messkanals (106) ist, und der mit einer Beschichtung bereitgestellt wird, die sich von der des Messkanals (106) unterscheidet oder ohne Beschichtung gebildet wird, und
nachdem die Flüssigkeitsfront der Probe in dem Negativkontrollkanal den Lesebereich erreicht hat, Beobachten oder Messen an den Lesebereichen der individuellen Kanäle ein Merkmal des Probenflusses, bevorzugt die Flussrate der Probe oder eine dazu proportionale Größe.

12. Verfahren nach Anspruch 11, wobei die Analyse der Immunreaktivität abzielt auf die Diagnose eines Zustandes oder einer Erkrankung begleitet von der Anwesenheit eines oder mehrerer spezifischer Antikörper, und der Fallenbereich von zumindest einem Messkanal ein Antigen aufweist, welches dazu fähig ist zumindest einen dieser spezifischen Antikörper zu binden, und wobei das Verfahren optional zusätzlich ein Vergleichen der in den individuellen Kanälen beobachteten oder gemessenen Flussmerkmale aufweist, wobei die Flussmerkmale bevorzugt die Flussraten oder dazu proportionale Größen sind.

13. Kapillarkanalsystem oder Verfahren nach einem der vorherigen Ansprüche, wobei das zumindest eine oder mehrere Antigene ausgewählt sind aus der Gruppe aufgelistet in Tabelle 1.

14. Kapillarkanalsystem oder Verfahren nach einem der vorherigen Ansprüche, wobei die Probe Vollblut ist.

15. Mikrofluidische Vorrichtung aufweisend ein Kapillarkanalsystem nach einem der Ansprüche 1 bis 10, wobei das Kapillarkanalsystem in dem Material oder den Materialien ausgebildet ist aus dem die Vorrichtung gebildet ist.

16. Verwendung des Kapillarkanalsystems nach einem der Ansprüche 1 bis 10, oder der mikrofluidische Vorrichtung nach Anspruch 15 als Schnelldiagnosevorrichtung für Point-of-Care serologische Untersuchungen.

## Revendications

1. Système de canal capillaire, qui comprend
- un canal de mesure (106), lequel canal de mesure (106) est un canal capillaire comprenant
∘ une section de piégeage (1, 22, 38, 40, 42, 44, 46, 47, 48) ayant un revêtement comprenant un antigène, la section de piégeage (1, 22, 38, 40, 42, 44, 46, 47, 48) permet l'adhésion de cellules d'un échantillon,
∘ une section de collecte (2, 20) ayant un volume qui est au moins aussi important que celui de la section de piégeage (1, 22, 38, 40, 42, 44, 46, 47, 48), et étant adaptée pour recevoir un échantillon s'écoulant à travers la section de piégeage (1, 22, 38, 40, 42, 44, 46, 47, 48), et
∘ une section de lecture (3, 18) adjacente à la section de collecte (2, 20),
où la section de collecte (2, 20) est située dans la partie entre la section de piégeage (1, 22, 38, 40, 42, 44, 46, 47, 48) et la section de lecture (3, 18) du canal capillaire, et
- un canal de commande négative (10, 104) présentant une configuration structurelle identique à celle du canal de mesure (106) et étant pourvu d'un revêtement qui est différent de celui du canal de mesure (106) ou étant formé sans revêtement.

2. Système de canal capillaire selon la revendication 1, **caractérisé en ce que** la section de piégeage (1, 22, 38, 40, 42, 44, 46, 47, 48) comprend au moins un passage ayant une surface de section transversale pouvant être traversée dont une taille caractéristique est comprise entre 1 et 20 micromètre(s), de préférence inférieure ou égale à 10 micromètres.

3. Système de canal capillaire selon la revendication 1 ou 2, **caractérisé en ce que** la section de piégeage (1, 22, 38, 40, 42, 44, 46, 47, 48) comprend un élément d'étranglement, et la surface de section transversale pouvant être traversée de l'au moins un passage est formée au moyen de l'au moins un élément d'étranglement.

4. Système de canal capillaire selon l'une des revendications 1 à 3, **caractérisé en ce que** la section de lecture (3, 18) a une paroi transparente.

5. Système de canal capillaire selon l'une des revendications 1 à 4, **caractérisé en ce que** le canal de commande négative (10, 104) qui - en cas d'écoulement du même échantillon - dans sa partie aval depuis la section de piégeage (1, 22, 38, 40, 42, 44, 46, 47, 48), fournit un débit supérieur ou égal à celui dans le canal de mesure.

6. Système de canal capillaire selon l'une des revendications 1 à 5, **caractérisé en ce qu'**il comprend un canal de commande positive (16) ayant une configuration structurelle identique à celle du canal de mesure (106) et comprenant un revêtement qui diffère de celui du canal de mesure, et - en cas d'écoulement du même échantillon - dans sa partie aval depuis la section de piégeage (1, 22, 38, 40, 42, 44, 46, 47, 48), fournissant un débit inférieur ou égal à celui dans le canal de mesure.

7. Système de canal capillaire selon l'une des revendications 1 à 6, **caractérisé en ce qu'**il comprend plus d'un canal de mesure de configurations structurelles identiques ou différentes.

8. Système de canal capillaire selon l'une des revendications 1 à 7, **caractérisé en ce qu'**il comprend plus d'un canal de commande négative de configurations structurelles identiques ou différentes.

9. Système de canal capillaire selon l'une des revendications 1 à 8, **caractérisé en ce qu'**il comprend plus d'un canal de commande positive de configurations structurelles identiques ou différentes.

10. Système de canal capillaire selon l'une des revendications 1 à 6, **caractérisé en ce qu'**il comprend un seul canal de mesure (106) et un seul canal de commande négative (104), et les points d'extrémité du canal de mesure et du canal de commande négative (104) sont reliés à un canal capillaire de drainage commun.

11. Procédé d'analyse de l'immunoréactivité comprenant le fait d'introduire un échantillon dans un système de canal capillaire qui comprend
- un canal de mesure (106), lequel canal de mesure (106) est un canal capillaire comprenant
∘ une section de piégeage (1, 22, 38, 40, 42, 44, 46, 47, 48) ayant un revêtement comprenant un antigène, la section de piégeage (1, 22, 38, 40, 42, 44, 46, 47, 48) permet l'adhésion de cellules d'un échantillon,
∘ une section de collecte (2, 20) ayant un volume qui est au moins aussi important que celui de la section de piégeage (1, 22, 38, 40, 42, 44, 46, 47, 48), et étant adaptée pour recevoir un échantillon s'écoulant à travers la section de piégeage (1, 22, 38, 40, 42, 44, 46, 47, 48), et
∘ une section de lecture (3, 18) adjacente à la section de collecte (2, 20),
où la section de collecte (2, 20) est située dans la partie entre la section de piégeage (1, 22, 38, 40, 42, 44, 46, 47, 48) et la section de lecture (3, 18) du canal capillaire, et
- un canal de commande négative (10, 104) présentant une configuration structurelle identique à celle du canal de mesure (106) et étant pourvu d'un revêtement qui est différent de celui du canal de mesure (106) ou étant formé sans revêtement, et
après que le début du liquide de l'échantillon dans le canal de commande négative a atteint la section de lecture, d'observer ou de mesurer sur les sections de lecture des canaux individuels une caractéristique de l'écoulement de l'échantillon, de préférence le débit de l'échantillon ou une quantité proportionnelle à celui-ci.

12. Procédé selon la revendication 11, dans lequel l'analyse de l'immunoréactivité est destinée au diagnostic d'une affection ou d'un trouble accompagné(e) de la présence d'un ou de plusieurs anticorps spécifique(s), et la section de piégeage d'au moins un canal de mesure comprend un antigène capable de se lier à au moins l'un desdits anticorps spécifiques, et le procédé comprend éventuellement en outre le fait de comparer les caractéristiques d'écoulement observées ou mesurées dans les canaux individuels, où les caractéristiques d'écoulement sont de préférence les débits ou les quantités proportionnelles à celui-ci.

13. Système de canal capillaire ou procédé selon l'une des revendications précédentes, dans lequel le ou les plusieurs antigène(s) est/sont choisi(s) parmi ceux listés dans le Tableau 1.

14. Système de canal capillaire ou procédé selon l'une des revendications précédentes, dans lequel l'échantillon est du sang total.

15. Dispositif microfluidique comprenant un système de canal capillaire selon l'une des revendications 1 à 10, dans lequel le système de canal capillaire est formé dans le matériau ou les matériaux constituant le dispositif.

16. Utilisation du système de canal capillaire selon l'une des revendications 1 à 10, ou du dispositif microfluidique selon la revendication 15 en tant que dispositif de diagnostic rapide pour des tests sérologiques à des points d'intervention.
